# FASCICULE DE BREVET EUROPEEN

(11) **EP 2 403 517 B1**
(45) Date de publication et mention de la délivrance du brevet: **17.04.2019**
(21) Numéro de dépôt: 10715294.4
(22) Date de dépôt: 04.03.2010
(51) Int. Cl.: A61K 38/17, A61P 29/00, A61K 31/18, G01N 33/569, A61K 45/06

(54) **NOUVEL AGENT ANTIVIRAL**
NEUES ANTIVIRUSMITTEL
NOVEL ANTIVIRAL AGENT

(30) Priorité: 04.03.2009 FR 0951347
(43) Date de publication de la demande: 11.01.2012
(73) Titulaire: Centre National de la Recherche Scientifique (C.N.R.S.), 75794 Paris Cedex 16 (FR); Université de Strasbourg, 67081 Strasbourg cedex (FR); Universite De La Mediterranee, 13284 Marseille Cedex 07 (FR)
(72) Inventeur: CHABRIERE, Eric, F-13009 Marseille (FR); ELIAS, Mikael, F-57190 Florange (FR); ROHR, Olivier, F-25480 Miserey-Salines (FR); SCHWARTZ, Christian, F-67380 Lingolsheim (FR)
(74) Mandataire: Lavoix
(86) Numéro de dépôt international: PCT/FR2010/050369
(87) Numéro de publication internationale: WO 2010/100381

(56) Documents cités:
- FR-A1- 2 861 731
- DARBINIAN-SARKISSIAN N ET AL: "p27(SJ), a novel protein in St John's Wort, that suppresses expression of HIV-1 genome", GENE THERAPY, vol. 13, no. 4, février 2006 (2006-02), pages 288-295, XP002561014, ISSN: 0969-7128
- PERERA ET AL: "Proteins related to St. John's Wort p27<SJ>, a suppressor of HIV-1 expression, are ubiquitous in plants", PHYTOCHEMISTRY, PERGAMON PRESS, GB, vol. 69, no. 4, 19 novembre 2007 (2007-11-19), pages 865-872, XP022473168, ISSN: 0031-9422
- MORALES R ET AL: "Serendipitous Discovery and X-Ray Structure of a Human Phosphate Binding Apolipoprotein", STRUCTURE, CURRENT BIOLOGY LTD., PHILADELPHIA, PA, US, vol. 14, no. 3, 1 mars 2006 (2006-03-01), pages 601-609, XP025134109, ISSN: 0969-2126 [extrait le 2006-03-01]

### SEQUENCE LISTING

<110> CENTRE NATIONAL DE LA RECHERCHE SCIENTIFIQUE UNIVERSITE DE STRASBOURG UNIVERSITE DE LA MEDITERRANEE AIX MARSEILLE II CHABRIERE, Eric ELIAS, Mikael ROHR, Olivier SCHWARTZ, Christian
<120> NOUVEL AGENT ANTIVIRAL
<130> IFB 09 AA CNR HPB2
<150> FR 09/51347
   <151> 2009-03-04
<160> 10
<170> PatentIn version 3.5
<210> 1
   <211> 376
   <212> PRT
   <213> Homo sapiens
<220>
   <221> MISC_FEATURE
   <222> (1)..(1)
   <223> D ou S
<220>
   <221> MISC_FEATURE
   <222> (3)..(3)
   <223> N ou D
<220>
   <221> MISC_FEATURE
   <222> (11)..(11)
   <223> Q ou E
<220>
   <221> MISC_FEATURE
   <222> (30)..(30)
   <223> V ou T
<220>
   <221> MISC_FEATURE
   <222> (43)..(43)
   <223> K ou S
<220>
   <221> MISC_FEATURE
   <222> (50)..(50)
   <223> D ou N
<220>
   <221> MISC_FEATURE
   <222> (54)..(54)
   <223> N ou D
<220>
   <221> MISC_FEATURE
   <222> (67)..(67)
   <223> T ou S
<220>
   <221> MISC_FEATURE
   <222> (68)..(68)
   <223> E ou Q
<220>
   <221> MISC_FEATURE
   <222> (75)..(75)
   <223> D ou N
<220>
   <221> MISC_FEATURE
   <222> (77)..(77)
   <223> E ou Q
<220>
   <221> MISC_FEATURE
   <222> (85)..(85)
   <223> Q ou E
<220>
   <221> MISC_FEATURE
   <222> (102)..(102)
   <223> A ou G
<220>
   <221> MISC_FEATURE
   <222> (122)..(122)
   <223> D ou N
<220>
   <221> MISC_FEATURE
   <222> (143)..(143)
   <223> S ou V
<220>
   <221> MISC_FEATURE
   <222> (219)..(219)
   <223> T ou S
<220>
   <221> MISC_FEATURE
   <222> (224)..(224)
   <223> D ou N
<220>
   <221> MISC_FEATURE
   <222> (252)..(252)
   <223> V ou S
<220>
   <221> MISC_FEATURE
   <222> (266)..(266)
   <223> D ou N
<400> 1
   Xaa Ile Xaa Gly Gly Gly Ala Thr Leu Pro Xaa Lys Leu Tyr Leu Thr
   1               5                   10                  15
   Pro Asp Val Leu Thr Ala Gly Phe Ala Pro Tyr Ile Gly Xaa Gly Ser
               20                  25                  30
   Gly Lys Gly Lys Ile Ala Phe Leu Glu Asn Xaa Tyr Asn Gln Phe Gly
           35                  40                  45
   Thr Xaa Thr Thr Lys Xaa Val His Trp Ala Gly Ser Asp Ser Lys Leu
       50                  55                  60
   Thr Ala Xaa Xaa Leu Ala Thr Tyr Ala Ala Xaa Lys Xaa Pro Gly Trp
   65                  70                  75                  80
   Gly Lys Leu Ile Xaa Val Pro Ser Val Ala Thr Ser Val Ala Ile Pro
                   85                  90                  95
   Phe Arg Lys Ala Gly Xaa Asn Ala Val Asp Leu Ser Val Lys Glu Leu
               100                 105                 110
   Cys Gly Val Phe Ser Gly Arg Ile Ala Xaa Trp Ser Gly Ile Thr Gly
           115                 120                 125
   Ala Gly Arg Ser Gly Pro Ile Gln Val Val Tyr Arg Ala Glu Xaa Ser
       130                 135                 140
   Gly Thr Thr Glu Leu Phe Thr Arg Phe Leu Asn Ala Lys Cys Thr Thr
   145                 150                 155                 160
   Gln Pro Gly Thr Phe Ala Val Thr Thr Val Phe Ala Asn Ser Tyr Ser
                   165                 170                 175
   Leu Gly Leu Ser Pro Leu Ala Gly Ala Val Ala Ala Ile Gly Ser Val
               180                 185                 190
   Gly Val Met Ala Ala Asp Asn Asp Val Thr Thr Ala Gln Gly Arg Ile
           195                 200                 205
   Thr Tyr Ile Ser Pro Asp Phe Ala Ala Pro Xaa Leu Ala Gly Leu Xaa
       210                 215                 220
   Asp Ala Thr Lys Val Ala Arg Thr Gly Lys Gly Ser Ser Ser Gly Gly
   225                 230                 235                 240
   Gly Ala Glu Gly Lys Ser Pro Ala Ala Ala Asn Xaa Ser Ala Ala Ile
                   245                 250                 255
   Ser Val Val Pro Leu Pro Ala Ala Ala Xaa Arg Gly Asp Pro Asn Val
                   260                 265                 270
   Trp Thr Pro Val Phe Gly Ala Val Thr Gly Gly Gly Val Val Ala Tyr
           275                 280                 285
   Pro Asp Ser Gly Tyr Pro Ile Leu Gly Phe Thr Asp Leu Ile Phe Ser
       290                 295                 300
   Glu Cys Tyr Ala Asn Ala Thr Gln Thr Gly Gln Val Arg Asn Phe Phe
   305                 310                 315                 320
   Thr Lys His Tyr Gly Thr Ser Ala Asn Asp Asn Ala Ala Ile Gln Ala
                   325                 330                 335
   Asn Ala Phe Val Pro Leu Pro Ser Asn Trp Lys Ala Ala Val Arg Ala
               340                 345                 350
   Ser Tyr Leu Thr Ala Ser Asn Ala Leu Ser Ile Gly Asp Ser Ala Val
           355                 360                 365
   Cys Gly Gly Lys Gly Arg Pro Glu
       370                 375
<210> 2
   <211> 376
   <212> PRT
   <213> Homo sapiens
<400> 2
   Asp Ile Asn Gly Gly Gly Ala Thr Leu Pro Gln Lys Leu Tyr Leu Thr
   1               5                   10                  15
   Pro Asp Val Leu Thr Ala Gly Phe Ala Pro Tyr Ile Gly Val Gly Ser
               20                  25                  30
   Gly Lys Gly Lys Ile Ala Phe Leu Glu Asn Lys Tyr Asn Gln Phe Gly
           35                  40                  45
   Thr Asp Thr Thr Lys Asn Val His Trp Ala Gly Ser Asp Ser Lys Leu
       50                  55                  60
   Thr Ala Thr Glu Leu Ala Thr Tyr Ala Ala Asp Lys Glu Pro Gly Trp
   65                  70                  75                  80
   Gly Lys Leu Ile Gln Val Pro Ser Val Ala Thr Ser Val Ala Ile Pro
                   85                  90                  95
   Phe Arg Lys Ala Gly Ala Asn Ala Val Asp Leu Ser Val Lys Glu Leu
               100                 105                 110
   Cys Gly Val Phe Ser Gly Arg Ile Ala Asp Trp Ser Gly Ile Thr Gly
           115                 120                 125
   Ala Gly Arg Ser Gly Pro Ile Gln Val Val Tyr Arg Ala Glu Ser Ser
       130                 135                 140
   Gly Thr Thr Glu Leu Phe Thr Arg Phe Leu Asn Ala Lys Cys Thr Thr
   145                 150                 155                 160
   Gln Pro Gly Thr Phe Ala Val Thr Thr Val Phe Ala Asn Ser Tyr Ser
                   165                 170                 175
   Leu Gly Leu Ser Pro Leu Ala Gly Ala Val Ala Ala Ile Gly Ser Val
               180                 185                 190
   Gly Val Met Ala Ala Asp Asn Asp Val Thr Thr Ala Gln Gly Arg Ile
           195                 200                 205
   Thr Tyr Ile Ser Pro Asp Phe Ala Ala Pro Thr Leu Ala Gly Leu Asp
       210                 215                 220
   Asp Ala Thr Lys Val Ala Arg Thr Gly Lys Gly Ser Ser Ser Gly Gly
   225                 230                 235                 240
   Gly Ala Glu Gly Lys Ser Pro Ala Ala Ala Asn Val Ser Ala Ala Ile
                   245                 250                 255
   Ser Val Val Pro Leu Pro Ala Ala Ala Asp Arg Gly Asp Pro Asn Val
               260                 265                 270
   Trp Thr Pro Val Phe Gly Ala Val Thr Gly Gly Gly Val Val Ala Tyr
           275                 280                 285
   Pro Asp Ser Gly Tyr Pro Ile Leu Gly Phe Thr Asp Leu Ile Phe Ser
       290                 295                 300
   Glu Cys Tyr Ala Asn Ala Thr Gln Thr Gly Gln Val Arg Asn Phe Phe
   305                 310                 315                 320
   Thr Lys His Tyr Gly Thr Ser Ala Asn Asp Asn Ala Ala Ile Gln Ala
                   325                 330                 335
   Asn Ala Phe Val Pro Leu Pro Ser Asn Trp Lys Ala Ala Val Arg Ala
               340                 345                 350
   Ser Tyr Leu Thr Ala Ser Asn Ala Leu Ser Ile Gly Asp Ser Ala Val
   355 360 365
   Cys Gly Gly Lys Gly Arg Pro Glu
   370 375
<210> 3
   <211> 376
   <212> PRT
   <213> Homo sapiens
<400> 3
   Ser Ile Asp Gly Gly Gly Ala Thr Leu Pro Glu Lys Leu Tyr Leu Thr
   1 5 10 15
   Pro Asp Val Leu Thr Ala Gly Phe Ala Pro Tyr Ile Gly Thr Gly Ser
   20 25 30
   Gly Lys Gly Lys Ile Ala Phe Leu Glu Asn Ser Tyr Asn Gln Phe Gly
   35 40 45
   Thr Asn Thr Thr Lys Asp Val His Trp Ala Gly Ser Asp Ser Lys Leu
   50 55 60
   Thr Ala Ser Gln Leu Ala Thr Tyr Ala Ala Asn Lys Gln Pro Gly Trp
   65 70 75 80
   Gly Lys Leu Ile Glu Val Pro Ser Val Ala Thr Ser Val Ala Ile Pro
   85 90 95
   Phe Arg Lys Ala Gly Gly Asn Ala Val Asp Leu Ser Val Lys Glu Leu
   100 105 110
   Cys Gly Val Phe Ser Gly Arg Ile Ala Asn Trp Ser Gly Ile Thr Gly
   115 120 125
   Ala Gly Arg Ser Gly Pro Ile Gln Val Val Tyr Arg Ala Glu Val Ser
   130 135 140
   Gly Thr Thr Glu Leu Phe Thr Arg Phe Leu Asn Ala Lys Cys Thr Thr
   145 150 155 160
   Gln Pro Gly Thr Phe Ala Val Thr Thr Val Phe Ala Asn Ser Tyr Ser
   165 170 175
   Leu Gly Leu Ser Pro Leu Ala Gly Ala Val Ala Ala Ile Gly Ser Val
   180 185 190
   Gly Val Met Ala Ala Asp Asn Asp Val Thr Thr Ala Gln Gly Arg Ile
   195 200 205
   Thr Tyr Ile Ser Pro Asp Phe Ala Ala Pro Ser Leu Ala Gly Leu Asn
   210 215 220
   Asp Ala Thr Lys Val Ala Arg Thr Gly Lys Gly Ser Ser Ser Gly Gly
   225 230 235 240
   Gly Ala Glu Gly Lys Ser Pro Ala Ala Ala Asn Ser Ser Ala Ala Ile
   245 250 255
   Ser Val Val Pro Leu Pro Ala Ala Ala Asn Arg Gly Asp Pro Asn Val
   260 265 270
   Trp Thr Pro Val Phe Gly Ala Val Thr Gly Gly Gly Val Val Ala Tyr
   275 280 285
   Pro Asp Ser Gly Tyr Pro Ile Leu Gly Phe Thr Asp Leu Ile Phe Ser
   290 295 300
   Glu Cys Tyr Ala Asn Ala Thr Gln Thr Gly Gln Val Arg Asn Phe Phe
   305 310 315 320
   Thr Lys His Tyr Gly Thr Ser Ala Asn Asp Asn Ala Ala Ile Gln Ala
   325 330 335
   Asn Ala Phe Val Pro Leu Pro Ser Asn Trp Lys Ala Ala Val Arg Ala
   340 345 350
   Ser Tyr Leu Thr Ala Ser Asn Ala Leu Ser Ile Gly Asp Ser Ala Val
   355 360 365
   Cys Gly Gly Lys Gly Arg Pro Glu
   370 375
<210> 4
   <211> 263
   <212> PRT
   <213> Hypericum perforatum
<400> 4
   Met Ala Asp Ile Asn Gly Gly Gly Ala Thr Leu Pro Gln Ala Leu Tyr
   1 5 10 15
   Gln Thr Ser Gly Val Leu Thr Ala Gly Phe Ala Pro Tyr Ile Gly Val
   20 25 30
   Gly Ser Gly Asn Gly Lys Ala Ala Phe Leu Asn Asn Asp Tyr Thr Lys
           35                  40                  45
   Phe Gln Ala Gly Val Thr Asn Lys Asn Val His Trp Ala Gly Ser Asp
       50                  55                  60
   Ser Lys Leu Ser Ala Thr Glu Leu Ser Thr Tyr Ala Ser Ala Lys Gln
   65                  70                  75                  80
   Pro Thr Trp Gly Lys Leu Ile Gln Val Pro Ser Val Gly Thr Ala Val
                   85                  90                  95
   Ala Ile Pro Phe Asn Lys Ser Gly Thr Ala Ala Val Asp Leu Ser Val
               100                 105                 110
   Ser Glu Leu Cys Gly Val Phe Ser Gly Arg Ile Thr Asp Trp Ser Gly
           115                 120                 125
   Ile Ser Gly Ser Gly Arg Thr Gly Ala Ile Thr Val Val Tyr Arg Ser
       130                 135                 140
   Glu Ser Ser Gly Thr Thr Glu Leu Phe Thr Arg Phe Leu Asn Ala Lys
   145                 150                 155                 160
   Cys Ala Glu Thr Gly Thr Phe Asn Ile Ser Thr Thr Phe Gly Thr Ser
                   165                 170                 175
   Tyr Thr Gly Gly Leu Pro Ala Gly Ala Val Ser Ala Ala Gly Ser Gln
               180                 185                 190
   Gly Val Met Thr Ala Leu Ala Gly Ala Asp Gly Gly Thr Thr Tyr Met
           195                 200                 205
   Ser Pro Asp Phe Ala Ala Pro Thr Leu Ala Gly Leu Asp Asp Ala Thr
       210                 215                 220
   Lys Val Ala Arg Val Gly Lys Asp Val Ala Thr Asn Thr Ala Gly Val
   225                 230                 235                 240
   Ser Pro Ala Ala Ala Asn Val Ser Ala Ala Ile Asn Ala Val Pro Val
                   245                 250                 255
   Pro Ala Ser Thr Glu Lys Pro
               260
<210> 5
   <211> 50
   <212> PRT
   <213> Homo sapiens
<220>
   <221> MISC_FEATURE
   <222> (1)..(1)
   <223> D ou S
<220>
   <221> MISC_FEATURE
   <222> (3)..(3)
   <223> N ou D
<220>
   <221> MISC_FEATURE
   <222> (11)..(11)
   <223> Q ou E
<220>
   <221> MISC_FEATURE
   <222> (30)..(30)
   <223> V ou T
<220>
   <221> MISC_FEATURE
   <222> (43)..(43)
   <223> K ou S
<220>
   <221> MISC_FEATURE
   <222> (50)..(50)
   <223> D ou N
<400> 5
   Xaa Ile Xaa Gly Gly Gly Ala Thr Leu Pro Xaa Lys Leu Tyr Leu Thr
   1               5                   10                  15
   Pro Asp Val Leu Thr Ala Gly Phe Ala Pro Tyr Ile Gly Xaa Gly Ser
               20                  25                  30
   Gly Lys Gly Lys Ile Ala Phe Leu Glu Asn Xaa Tyr Asn Gln Phe Gly
           35                  40                  45
   Thr Xaa
       50
<210> 6
   <211> 260
   <212> PRT
   <213> Homo sapiens
<220>
   <221> MISC_FEATURE
   <222> (1)..(1)
   <223> D ou S
<220>
   <221> MISC_FEATURE
   <222> (3)..(3)
   <223> N ou D
<220>
   <221> MISC_FEATURE
   <222> (11)..(11)
   <223> Q ou E
<220>
   <221> MISC_FEATURE
   <222> (30)..(30)
   <223> V ou T
<220>
   <221> MISC_FEATURE
   <222> (43)..(43)
   <223> K ou S
<220>
   <221> MISC_FEATURE
   <222> (50)..(50)
   <223> D ou N
<220>
   <221> MISC_FEATURE
   <222> (54)..(54)
   <223> N ou D
<220>
   <221> MISC_FEATURE
   <222> (67)..(67)
   <223> T ou S
<220>
   <221> MISC_FEATURE
   <222> (68)..(68)
   <223> E ou Q
<220>
   <221> MISC_FEATURE
   <222> (75)..(75)
   <223> D ou N
<220>
   <221> MISC_FEATURE
   <222> (77)..(77)
   <223> E ou Q
<220>
   <221> MISC_FEATURE
   <222> (85)..(85)
   <223> Q ou E
<220>
   <221> MISC_FEATURE
   <222> (102)..(102)
   <223> A ou G
<220>
   <221> MISC_FEATURE
   <222> (122)..(122)
   <223> D ou N
<220>
   <221> MISC_FEATURE
   <222> (143)..(143)
   <223> S ou V
<220>
   <221> MISC_FEATURE
   <222> (219)..(219)
   <223> T ou S
<220>
   <221> MISC_FEATURE
   <222> (224)..(224)
   <223> D ou N
<220>
   <221> MISC_FEATURE
   <222> (252)..(252)
   <223> V ou S
<400> 6
   Xaa Ile Xaa Gly Gly Gly Ala Thr Leu Pro Xaa Lys Leu Tyr Leu Thr
   1               5                   10                  15
   Pro Asp Val Leu Thr Ala Gly Phe Ala Pro Tyr Ile Gly Xaa Gly Ser
               20                  25                  30
   Gly Lys Gly Lys Ile Ala Phe Leu Glu Asn Xaa Tyr Asn Gln Phe Gly
           35                  40                  45
   Thr Xaa Thr Thr Lys Xaa Val His Trp Ala Gly Ser Asp Ser Lys Leu
       50                  55                  60
   Thr Ala Xaa Xaa Leu Ala Thr Tyr Ala Ala Xaa Lys Xaa Pro Gly Trp
   65                  70                  75                  80
   Gly Lys Leu Ile Xaa Val Pro Ser Val Ala Thr Ser Val Ala Ile Pro
                   85                  90                  95
   Phe Arg Lys Ala Gly Xaa Asn Ala Val Asp Leu Ser Val Lys Glu Leu
               100                 105                 110
   Cys Gly Val Phe Ser Gly Arg Ile Ala Xaa Trp Ser Gly Ile Thr Gly
           115                 120                 125
   Ala Gly Arg Ser Gly Pro Ile Gln Val Val Tyr Arg Ala Glu Xaa Ser
       130                 135                 140
   Gly Thr Thr Glu Leu Phe Thr Arg Phe Leu Asn Ala Lys Cys Thr Thr
   145                 150                 155                 160
   Gln Pro Gly Thr Phe Ala Val Thr Thr Val Phe Ala Asn Ser Tyr Ser
                   165                 170                 175
   Leu Gly Leu Ser Pro Leu Ala Gly Ala Val Ala Ala Ile Gly Ser Val
               180                 185                 190
   Gly Val Met Ala Ala Asp Asn Asp Val Thr Thr Ala Gln Gly Arg Ile
           195                 200                 205
   Thr Tyr Ile Ser Pro Asp Phe Ala Ala Pro Xaa Leu Ala Gly Leu Xaa
       210                 215                 220
   Asp Ala Thr Lys Val Ala Arg Thr Gly Lys Gly Ser Ser Ser Gly Gly
   225                 230                 235                 240
   Gly Ala Glu Gly Lys Ser Pro Ala Ala Ala Asn Xaa Ser Ala Ala Ile
                   245                 250                 255
   Ser Val Val Pro
               260
<210> 7
   <211> 50
   <212> PRT
   <213> Homo sapiens
<400> 7
   Asp Ile Asn Gly Gly Gly Ala Thr Leu Pro Gln Lys Leu Tyr Leu Thr
   1               5                   10                  15
   Pro Asp Val Leu Thr Ala Gly Phe Ala Pro Tyr Ile Gly Val Gly Ser
               20                  25                  30
   Gly Lys Gly Lys Ile Ala Phe Leu Glu Asn Lys Tyr Asn Gln Phe Gly
           35                  40                  45
   Thr Asp
       50
<210> 8
   <211> 260
   <212> PRT
   <213> Homo sapiens
<400> 8
   Asp Ile Asn Gly Gly Gly Ala Thr Leu Pro Gln Lys Leu Tyr Leu Thr
   1               5                   10                  15
   Pro Asp Val Leu Thr Ala Gly Phe Ala Pro Tyr Ile Gly Val Gly Ser
               20                  25                  30
   Gly Lys Gly Lys Ile Ala Phe Leu Glu Asn Lys Tyr Asn Gln Phe Gly
           35                  40                  45
   Thr Asp Thr Thr Lys Asn Val His Trp Ala Gly Ser Asp Ser Lys Leu
       50                  55                  60
   Thr Ala Thr Glu Leu Ala Thr Tyr Ala Ala Asp Lys Glu Pro Gly Trp
   65                  70                  75                  80
   Gly Lys Leu Ile Gln Val Pro Ser Val Ala Thr Ser Val Ala Ile Pro
                   85                  90                  95
   Phe Arg Lys Ala Gly Ala Asn Ala Val Asp Leu Ser Val Lys Glu Leu
               100                 105                 110
   Cys Gly Val Phe Ser Gly Arg Ile Ala Asp Trp Ser Gly Ile Thr Gly
           115                 120                 125
   Ala Gly Arg Ser Gly Pro Ile Gln Val Val Tyr Arg Ala Glu Ser Ser
       130                 135                 140
   Gly Thr Thr Glu Leu Phe Thr Arg Phe Leu Asn Ala Lys Cys Thr Thr
   145                 150                 155                 160
   Gln Pro Gly Thr Phe Ala Val Thr Thr Val Phe Ala Asn Ser Tyr Ser
                   165                 170                 175
   Leu Gly Leu Ser Pro Leu Ala Gly Ala Val Ala Ala Ile Gly Ser Val
               180                 185                 190
   Gly Val Met Ala Ala Asp Asn Asp Val Thr Thr Ala Gln Gly Arg Ile
           195                 200                 205
   Thr Tyr Ile Ser Pro Asp Phe Ala Ala Pro Thr Leu Ala Gly Leu Asp
       210                 215                 220
   Asp Ala Thr Lys Val Ala Arg Thr Gly Lys Gly Ser Ser Ser Gly Gly
   225                 230                 235                 240
   Gly Ala Glu Gly Lys Ser Pro Ala Ala Ala Asn Val Ser Ala Ala Ile
                   245                 250                 255
   Ser Val Val Pro
               260
<210> 9
   <211> 50
   <212> PRT
   <213> Homo sapiens
<400> 9
   Ser Ile Asp Gly Gly Gly Ala Thr Leu Pro Glu Lys Leu Tyr Leu Thr
   1               5                   10                  15
   Pro Asp Val Leu Thr Ala Gly Phe Ala Pro Tyr Ile Gly Thr Gly Ser
               20                  25                  30
   Gly Lys Gly Lys Ile Ala Phe Leu Glu Asn Ser Tyr Asn Gln Phe Gly
           35                  40                  45
   Thr Asn
       50
<210> 10
   <211> 260
   <212> PRT
   <213> Homo sapiens
<400> 10
   Ser Ile Asp Gly Gly Gly Ala Thr Leu Pro Glu Lys Leu Tyr Leu Thr
   1               5                   10                  15
   Pro Asp Val Leu Thr Ala Gly Phe Ala Pro Tyr Ile Gly Thr Gly Ser
               20                  25                  30
   Gly Lys Gly Lys Ile Ala Phe Leu Glu Asn Ser Tyr Asn Gln Phe Gly
           35                  40                  45
   Thr Asn Thr Thr Lys Asp Val His Trp Ala Gly Ser Asp Ser Lys Leu
       50                  55                  60
   Thr Ala Ser Gln Leu Ala Thr Tyr Ala Ala Asn Lys Gln Pro Gly Trp
   65                  70                  75                  80
   Gly Lys Leu Ile Glu Val Pro Ser Val Ala Thr Ser Val Ala Ile Pro
                   85                  90                  95
   Phe Arg Lys Ala Gly Gly Asn Ala Val Asp Leu Ser Val Lys Glu Leu
               100                 105                 110
   Cys Gly Val Phe Ser Gly Arg Ile Ala Asn Trp Ser Gly Ile Thr Gly
           115                 120                 125
   Ala Gly Arg Ser Gly Pro Ile Gln Val Val Tyr Arg Ala Glu Val Ser
           130                 135                 140
   Gly Thr Thr Glu Leu Phe Thr Arg Phe Leu Asn Ala Lys Cys Thr Thr
   145                 150                 155                 160
   Gln Pro Gly Thr Phe Ala Val Thr Thr Val Phe Ala Asn Ser Tyr Ser
                   165                 170                 175
   Leu Gly Leu Ser Pro Leu Ala Gly Ala Val Ala Ala Ile Gly Ser Val
               180                 185                 190
   Gly Val Met Ala Ala Asp Asn Asp Val Thr Thr Ala Gln Gly Arg Ile
           195                 200                 205
   Thr Tyr Ile Ser Pro Asp Phe Ala Ala Pro Ser Leu Ala Gly Leu Asn
       210                 215                 220
   Asp Ala Thr Lys Val Ala Arg Thr Gly Lys Gly Ser Ser Ser Gly Gly
   225                 230                 235                 240
   Gly Ala Glu Gly Lys Ser Pro Ala Ala Ala Asn Ser Ser Ala Ala Ile
                   245                 250                 255
   Ser Val Val Pro
               260

## Description

La présente invention décrit un nouvel agent antiviral, en particulier dirigé contre le virus de l'immunodéficience humaine VIH.

Le SIDA (Syndrome d'Immuno Déficience Acquis) correspond à l'une des plus grandes pandémies humaines. Le SIDA n'est pas une maladie à proprement dit, mais un ensemble de maladies liées à des infections opportunistes. Ces infections opportunistes peuvent se développer dans l'organisme du fait de la diminution des défenses immunitaires des personnes malades.

Le SIDA est causé par l'infection par un rétrovirus de la famille des lentivirus : le Virus de l'Immunodéficience Humaine (VIH), isolé dans les années 80 [Barré-Sinoussi F, et al, 1983, Science, May 20;220(4599):868-71 *;* de Clavel et al., Science, 1986 Jul 18;233(4761):343-6]. On dénombre deux type de virus VIH : les virus VIH 1 et VIH 2.

Le VIH affaiblit le système immunitaire en infectant les cellules présentatrices de l'antigène CD 4: les lymphocytes T CD4+, les macrophages, les cellules dendritiques et les cellules microgliales cérébrales. L'infection des cellules T CD4+, cellules « coordinatrices » de la réponse immunitaire acquise, est cruciale dans le développement de la maladie.

L'infection par le VIH évolue en plusieurs phases pouvant se succéder dans le temps :
1. la primo-infection avec (50 à 75 % des cas) ou sans symptômes ; c'est la phase de séroconversion qui suit la contamination.
2. une phase de latence, parfois accompagnée d'un état de lymphadénopathie généralisée,
3. une phase à symptômes mineurs de l'infection à virus de l'immunodéficience humaine,
4. la phase d'immunodépression profonde ou stade de Sida généralement symptomatique.

Durant les différentes phases, le taux de cellules T CD4 ne cesse de diminuer, du fait de la destruction des cellules T CD4, infectées ou non infectées.

La mort des cellules infectées est consécutive au détournement de la machinerie cellulaire des lymphocytes, qui ne peuvent plus fabriquer leurs propres protéines, ainsi qu'à la destruction de l'intégrité membranaire au moment de la sortie des virus neo-formés.

De plus, les cellules infectées exposent à leur surface membranaire des protéines virales (complexe Env), protéines reconnues par des cellules immunitaires saines qui s'accolent au lymphocyte infecté. De cette interaction s'ensuit un processus apoptotique induisant la mort des cellules saines.

A ce jour, il n'existe aucun traitement pour guérir l'infection au VIH. En effet, une personne séropositive infectée par le VIH, reste séropositive à vie.

Cependant, il existe de nombreux traitements capables de bloquer l'évolution du virus dans l'organisme et de maintenir l'équilibre entre la présence du virus et sa multiplication, et le maintien d'un système immunitaire fonctionnel.

Les traitements proposés aux patients séropositifs pour le VIH visent à inhiber les grandes étapes du cycle vital du virus.

L'infection par le VIH comprend les étapes suivantes :
- la fusion entre la cellule hôte et le virus, et la pénétration,
- la transcription inverse de l'ARN viral en ADN, via la transcriptase inverse
- l'intégration du génome viral dans le génome de la cellule hôte, via l'intégrase
- la transcription des gènes viraux, utilisant la machinerie cellulaire et la protéine transactivatrice Tat.
- la synthèse des protéines virales et leur maturation, via la protéase virale
- la formation des néo-virus.

Les antirétroviraux constituent l'arsenal thérapeutique contre le VIH, qui s'étoffe progressivement. Une vingtaine de médicaments antirétroviraux sont disponibles sur le marché et ont pour but d'interférer avec différents mécanismes, soit en inhibant les enzymes du VIH nécessaires à sa réplication, soit en bloquant les mécanismes d'entrée dans la cellule cible.

Les antiviraux communément utilisés dans le traitement de l'infection par le VIH ont pour cible la transcriptase inverse ou la protéase virale.

La transcriptase inverse peut être inhibée par différentes classes de molécules : les Inhibiteurs nucléosidiques (INTI), les Inhibiteurs non nucléosidiques (INNTI) et les Analogues nucléotidiques (IANTI).

Les INTI ont été les premiers antiviraux utilisés pour traiter le VIH à partir de 1985. Ils comprennent notamment la zidovudine (AZT), la didanosine (ddI), la zalcitabine (ddC), la stavudine (d4T), la lamivudine (3TC), l'abacavir (ABC) et l'emtricitabine (FTC). Il existe également un INTI modifié, l'IANTI correspondant au ténofovir.

Les mutations du génome du virus générées par la faible fidélité de transcription inverse de la transcriptase inverse confèrent au VIH une résistance aux INTI. Cette résistance peut être croisée entre plusieurs INTI.

Les INNTI sont des inhibiteurs puissants et très sélectifs de la transcriptase inverse du VIH, mais ne ciblent que le VIH-1. La névirapine, la delavirdine et l'efavirenz sont les trois INNTI les plus utilisés.

La protéase peut être inhibée par plusieurs molécules puissantes, efficaces sur les deux types de virus, et dont l'effet est de conduire à la formation de virus incapables d'infecter d'autres cellules cibles. Parmi ces molécules, on peut citer l'Atazanavir, le Fosamprenavir, le Lopinavir, le Darunavir, le Nelfinavir, le Ritonavir, le Saquinavir ou encore le Tipranavir. D'autres antiviraux visant à inhiber l'entrée du virus (inhibiteur d'entrée), l'intégration du génome viral dans la cellule hôte (inhibiteur d'intégrase) ou encore des inhibiteurs de maturation des protéines virales (inhibiteurs de maturations) ont été mis sur le marché.

Les trithérapies ou multi thérapies, associant inhibiteurs nucléosidiques (INTI), non nucléosidiques (INNTI) et/ou antiprotéases (IP), permettent une réduction de la charge virale en dessous des seuils de détectabilité chez un grand nombre de patients séropositifs au VIH. Cette efficacité a permis une diminution très notable de la mortalité liée à l'infection à VIH. Malheureusement, des génotypes indiquant une résistance à un antiviral ont été trouvés chez 80 % des patients et, de manière plus inquiétante, 45,5 % des populations virales sont résistantes à une association INTI/IP et 26 % à une association de trois classes d'anti-VIH (Tamalet et al, AIDS. 2003 Nov 7;17(16):2383-8).

Ce constat est particulièrement préoccupant lorsque l'on observe des effets secondaires liés à une prise au long terme des trithérapies (lipoatrophies, lipodystrophies, hypertriglycidémie, hypercholestérolémie, neuropathies...), observés chez 70 % des patients sous traitement, entraînant une mauvaise observance et des arrêts "sauvages" souvent à l'origine de l'apparition de résistances.

La mise au point de traitements moins contraignants, entraînant moins d'effets secondaires et ne présentant pas de profil de résistances croisées reste donc une priorité, malgré le grand nombre de médicaments actuellement commercialisés. Dans ce but, il est indispensable de cibler d'autres étapes du cycle réplicatif du VIH.

La protéine HPBP (human phoshate binding protein) est une protéine plasmatique qui fut découverte fortuitement lors d'études structurales sur la paraoxonase humaine [Morales, R., et al. (2006) Structure 14: 601-9].

La séquence en acides aminés relie HPBP à une famille de protéine nommé protéines DING, à cause de leur extrémité N-terminale très conservée [Diemer, H et al. (2008) Proteins 71: 1708-20]*.* Les protéines DING, bien qu'ubiquitaires chez les eucaryotes, sont étonnamment absentes des banques de données nucléotidiques [Berna, AF. Et al. (2008) Int J Biochem Cell Biol 40: 170-5].

Associée physiologiquement à la paraoxonase humaine, une protéine clairement impliquée dans le processus de l'athérosclérose [Shih, DM et al. (1998) Nature 394: 284-7], et capable de fixer le phosphate inorganique, HPBP est impliquée dans l'athérosclérose et utilisée comme marqueur du risque cardio-vasculaire (WO 2005/042572).

La structure de la HPBP a été élucidée [Morales, R., et al. (2006) Structure 14: 601-9], et il a été montré que cette protéine est capable de lier le phosphate inorganique (Pi).

La HPBP et la paraoxonase PON-1 sont étroitement associées, et cette association mutuelle conduit à leur stabilisation réciproque [Rochu D et al. Toxicology. 2007 Apr 20;233(1-3):47-59*,* Rochu D. et al. Biochim Biophys Acta. 2007 Jul;1774(7):874-83*,* Rochu et al. Biochem Soc Trans. 2007 Dec;35(Pt 6):1616-20*].*

A ce jour, la séquence nucléotidique de la protéine humaine HPBP n'a pas été isolée.

Un des buts de l'invention est de fournir un nouveau traitement pour lutter contre les infections virales.

Un autre but de l'invention est de fournir de nouvelles compositions pharmaceutiques antivirales présentant des effets secondaires réduits, et mieux tolérées par les patients. Egalement, un des buts de l'invention est de fournir de nouvelles compositions antivirales agissant sur d'autres cibles du cycle viral.

La présente invention décrit l'utilisation d'une composition comprenant ou constituée par au moins un élément choisi parmi :
- une protéine déterminée comprenant la séquence d'acides aminés SEQ ID NO : 5,
- une protéine homologue de ladite protéine déterminée, possédant une séquence d'acides aminés présentant une homologie d'au moins 72% avec la séquence SEQ ID NO : 5, sous réserve que lesdites protéines précédentes interagissent avec le facteur de transcription C/EBPβ et / ou la protéine virale Tat, pour la préparation d'un médicament destiné à la prévention ou au traitement d'une infection virale.

La présente invention concerne l'utilisation d'une composition comprenant ou constituée par au moins un élément choisi parmi :
- une protéine déterminée comprenant ou constituée de la séquence d'acides aminés SEQ ID NO : 5, et
- une protéine homologue de ladite protéine déterminée, possédant une séquence d'acides aminés présentant une homologie d'au moins 72% avec la séquence SEQ ID NO : 5, sous réserve que lesdites protéines précédentes interagissent avec le facteur de transcription C/EBPβ et / ou la protéine virale Tat,
   pour
   - la préparation d'un médicament destiné à la prévention ou au traitement de pathologies associées à une infection virale ou à une inflammation, sous réserve que, dans le cadre des pathologies liées à l'inflammation, ledit élément ne soit pas l'une quelconque des protéines SEQ ID NO : 1 à 3, ou
   - pour la mise en oeuvre d'une méthode *in vitro,* de mesure la susceptibilité à une infection virale

Dans un mode de réalisation avantageux, l'invention concerne un concerne l'utilisation d'une composition comprenant ou constituée par au moins un élément choisi parmi :
- une protéine déterminée comprenant ou constituée de la séquence d'acides aminés SEQ ID NO : 5, et
- une protéine homologue de ladite protéine déterminée, possédant une séquence d'acides aminés présentant une homologie d'au moins 72% avec la séquence SEQ ID NO : 5,
sous réserve que lesdites protéines précédentes interagissent avec le facteur de transcription C/EBPβ et / ou la protéine virale Tat, pour la préparation d'un médicament destiné à la prévention ou au traitement d' une infection virale.

Un mode de réalisation avantageux de l'invention décrit l'utilisation d'une composition telle que définie précédemment, où ladite protéine déterminée est constituée par une séquence d'acides aminés choisie parmi les séquences d'acides aminés suivantes SEQ ID NO : 5, SEQ ID NO : 7 et SEQ ID NO : 9.

Un mode de réalisation avantageux de l'invention décrit l'utilisation d'une composition telle que définie précédemment, comprenant ou constituée par au moins un élément choisi parmi :
- une protéine déterminée comprenant ou constituée de la séquence d'acides aminés SEQ ID NO : 6, et
- une protéine homologue de ladite protéine déterminée, possédant une séquence d'acides aminés présentant une homologie d'au moins 72% avec la séquence SEQ ID NO : 6, sous réserve que lesdites protéines précédentes interagissent avec le facteur de transcription C/EBPβ et / ou la protéine virale Tat.

Un mode de réalisation avantageux de l'invention décrit l'utilisation d'une composition telle que définie précédemment, où ladite protéine déterminée est constituée par une séquence d'acides aminés choisie parmi les séquences d'acides aminés suivantes SEQ ID NO : 6, SEQ ID NO : 8 et SEQ ID NO : 10.

Un mode de réalisation avantageux de l'invention décrit l'utilisation d'une composition telle que définie précédemment, comprenant ou constituée par au moins un élément choisi parmi :
- une protéine déterminée comprenant ou constituée de la séquence d'acides aminés SEQ ID NO : 1, et
- une protéine homologue de ladite protéine déterminée, possédant une séquence d'acides aminés présentant une homologie d'au moins 72% avec la séquence SEQ ID NO : 1 sous réserve que lesdites protéines précédentes interagissent avec le facteur de transcription C/EBPβ et / ou la protéine virale Tat.

Un mode de réalisation avantageux de l'invention décrit l'utilisation d'une composition telle que définie précédemment, où ladite protéine déterminée est constituée par une séquence d'acides aminés choisie parmi les séquences d'acides aminés suivantes SEQ ID NO : 1, SEQ ID NO : 2 et SEQ ID NO : 3.

Un mode de réalisation avantageux de l'invention décrit l'utilisation d'une composition telle que définie précédemment, où ladite protéine déterminée est constituée par une séquence d'acides aminés choisie parmi les séquences d'acides aminés suivantes SEQ ID NO : 1, SEQ ID NO : 2 et SEQ ID NO : 3, pour la préparation d'un médicament dans le cadre du traitement d'infection virale.

La présente invention repose sur la mise en évidence inattendue par les Inventeurs de propriétés antivirales de la protéine HPBP, et plus particulièrement d'une protéine comprenant la séquence SEQ ID NO : 5.

La séquence SEQ ID NO : 5 correspond aux 50 premiers acides aminés de la séquence SEQ ID NO : 1 définie ci après.

La séquence SEQ ID NO : 6 correspond aux 260 premiers acides aminés de la séquence SEQ ID NO : 1 définie ci-après.

Plus précisément, l'invention repose sur la démonstration faite par les Inventeur de l'action inhibitrice de la protéine HPBP, ou de ses fragments, sur la transcription, notamment des gènes viraux ou de gènes impliqués dans l'inflammation, nécessitant le facteur de transcription C/EBPβ.

Dans l'invention, il est appelé «protéine déterminée », toute protéine, peptide, fragment de protéines ou de peptides, ou connu dont la synthèse à lieu au sein d'un organisme vivant, généralement sans intervention extérieure humaine, et isolée de son contexte naturel.

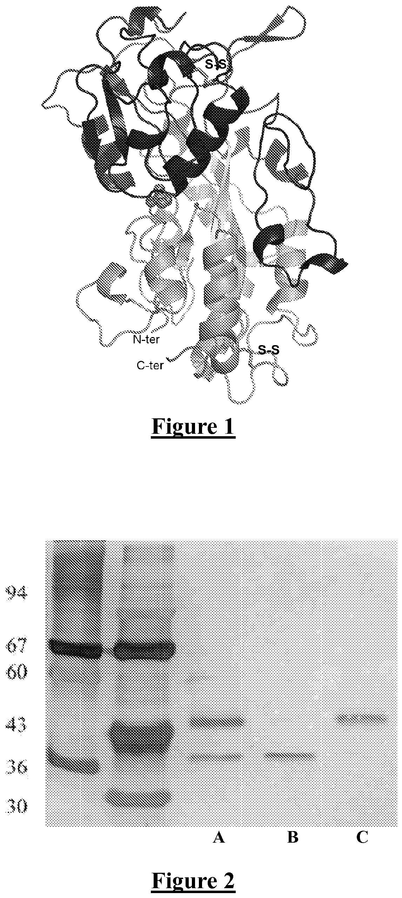

La protéine HPBP peut être définie par sa séquence d'acides aminés SEQ ID NO : 1. La structure tridimensionnelle de la HPBP est représentée sur la figure 1.

Dans l'invention « une protéine homologue à ladite protéine déterminée », correspond à une protéine dérivée de ladite protéine déterminée, ladite protéine dérivée étant obtenue par mutation, suppression ou addition d'un ou plusieurs acides aminés à la séquence de ladite protéine déterminée.

Plus particulièrement, « une protéine homologue à ladite protéine déterminée » est définie dans l'invention comme une protéine obtenue par substitution d'un ou plusieurs acides aminés sous réserve que ladite protéine homologue présente au moins 72% d'homologie avec la séquence SEQ ID NO : 5, et qu'elle possède la séquence suivante :
D-I-N-G-G-G-Xn-F-G, où X représente l'un quelconque des 20 acides aminés naturels, et n varie de environ 30 à environ 40, notamment 40.

Les données d'interaction entre la protéine Tat et la protéine HPBP sont présentées dans la partie Exemples ci-après.

De ces données, l'homme de métier peut retrouver les acides aminés minimaux essentiels permettant l'association HPBP/Tat.

Dans l'invention, on entend par « infection virale » toute maladie déclenchée par un virus et la propagation de ce virus dans l'organisme (et aux dépens de l'organisme). La mesure d'une infection virale est réalisée communément par la détection dans l'organisme infecté de marqueurs viraux spécifiques. Par exemple, pour le VIH, le marqueur p24 est un témoin de l'infection. Le VIH peut également être détecté par la mesure de la charge virale, c'est-à-dire la quantité d'ARN viral.

Par « inflammation », il est défini dans la description une réaction de défense immunitaire stéréotypée de l'organisme mettant en jeu, entre autre, une vasodilation. Entre autres mécanismes, l'inflammation est caractérisée par la sécrétion de facteurs pro-inflammatoires tels que l'IL-6, le TNF-α ou la chimokine MCP-1. Une mesure de l'inflammation peut être réalisée par le dosage des facteurs pro-inflammatoires susmentionnés.

Dans la description, « les pathologies associées à l'inflammation » peuvent être représentées par l'arthrite rhumatoïde, maladies auto-immunes et notamment le lupus, plus particulièrement le lupus érythémateux disséminé, allergies alimentaires ou allergies des voies respiratoires, notamment les allergies au pollen, le syndrome de Sjögren, la sclérodermie (sclérose systémique), la dermatomyosite, la polymyosite, la polymyalgia rheumatica, l'ostéoarthrite, l'arthrite septique ou encore la vascularite.

La protéine p27^{SJ} de millepertuis, représentée par la séquence d'acides aminés SEQ ID NO : 4, est exclue de l'invention car elle présente une homologie de séquence de 71% avec la séquence SEQ ID NO : 1.

Les termes « interagisse avec le facteur de transcription C/EBPβ et / ou la protéine virale Tat » signifient qu'il peut exister une interaction avec la protéine C/EBPβ seule ou avec la protéine Tat seule, ou encore qu'il existe une interaction avec la protéine C/EBPβ et avec la protéine Tat. Les termes susmentionnés peuvent donc être interprétés comme suit : « interagisse avec le facteur de transcription C/EBPβ, ou interagisse avec la protéine virale Tat ou encore interagisse avec le facteur de transcription C/EBPβ et la protéine virale Tat ».

Les termes « interagisse avec le facteur de transcription C/EBPβ » ont pour signification dans l'invention qu'il existe une liaison directe, chimique, électrostatique ou ionique, entre une protéine, homologue ou variant de la protéine HPBP, et le facteur de transcription C/EBPβ. Cette interaction peut être révélée par des méthodes classiques connues de l'homme de métier, telles que la co-immunoprécipitation, ou encore un test ELISA tel que défini ci-après, lesdites techniques n'étant pas limitatives.

Les termes « interagisse avec la protéine Tat » ont pour signification dans l'invention qu'il existe une liaison directe, chimique, électrostatique ou ionique, entre une protéine, homologue ou variant de la protéine HPBP, et la protéine Tat. Cette interaction peut être révélée par des méthodes classiques connues de l'homme de métier, telles que la co-immunoprécipitation, ou encore un test ELISA tel que défini ci-après, lesdites techniques n'étant pas limitatives

La famille C/EBP (CCAAT-enhancer-binding proteins) est constituée d'un nombre important de facteurs de transcription de type "leucine-zipper". Ces facteurs sont des protéines capables de se lier sur des motifs d'ADN riches en motif CCAAT, lesdits motifs CCAAT étant présents dans la séquence des promoteurs de nombreux gènes, et de réguler l'expression des gènes. Ces facteurs sont connus pour jouer un rôle important dans des fonctions spécialisées des hépatocytes, adipocytes et macrophages [Li H, et al. Cell Mol Immunol ; 2007 dec ; 4(6):407-18].

Des motifs de type CCAAT ont été décrits soit dans les promoteurs de gènes viraux, soit dans les longues séquences terminale répétées (LTR) des rétrovirus. La présence des sites CCAAT et la fixation du facteur C/EBPβ en particulier a pour conséquence d'induire l'expression des gènes sous contrôle de ces séquences régulatrices. On peut citer comme exemple de virus, sans pour autant être limitatif, les virus VIH, les virus de l'hépatite, en particulier l'hépatite B, les papillomavirus humains, les adénovirus dont l'adénovirus Ad-36 et les virus de l'herpès. [Kinoshita SM, et al. Proc Natl Acad Sci USA; 2008 Sep 30;105(39):15022-7 *;* Wooldridge TR, et al. Virology Volume 374, Issue 2, 10 May 2008, Pages 371-380*;* Ralph WM Jr et al.; J Gen Virol. 2006 Jan;87(Pt 1):51-9*;* Rogers PM, et al.; Int J Obes (Lond). 2008 Mar;32(3):397-406].
C/EBPβ, également appelé NF-IL6 (Nuclear Factor IL-6) joue un rôle important dans la régulation des gènes impliqués dans la réponse immune et inflammatoire, et notamment interagit avec l'élément de réponse à l'IL-1 du promoteur du gène pro-inflammatoire IL-6. C/EBPβ a également été montré comme étant capable d'induire l'expression des gènes du TNFα ou du facteur chimioattractant des monocytes MCP-1.

La séquence SEQ ID NO : 2 correspond à la séquence d'acides aminés de la protéine HPBP humaine, possédant des propriétés de liaison au phosphate et qui a été isolée du plasma humain. Le protocole de purification de la HPBP possédant une séquence d'acides aminés définie consistant en la séquence SEQ ID NO : 2 est détaillé dans la demande internationale WO 2005/042572. Cette méthode de purification est basée sur sa co-purification avec la paraoxonase humaine PON-1. La purification de HPBP du complexe HPBP/PON-1 est réalisée selon le protocole décrit dans Renault et al. [Renault et al. J Chromatogr B Analyt Technol Biomed Life Sci. 2006 May 19;836(1-2):15-21]. Le protocole détaillé est décrit dans la section Exemple ci-après.

La séquence SEQ ID NO : 3 correspond à la séquence d'acides aminés d'une protéine présentant une identité d'environ 90% avec la protéine HPBP humaine, et possédant des propriétés de liaison au phosphate, c'est-à-dire des propriétés de fixation du phosphate inorganique.

La séquence SEQ ID NO : 7 correspond aux 50 premiers acides aminés de la séquence SEQ ID NO : 2.

La séquence SEQ ID NO : 8 correspond aux 260 premiers acides aminés de la séquence SEQ ID NO : 2.

La séquence SEQ ID NO : 9 correspond aux 50 premiers acides aminés de la séquence SEQ ID NO : 3.

La séquence SEQ ID NO : 10 correspond aux 260 premiers acides aminés de la séquence SEQ ID NO : 3.

Les propriétés de liaison/fixation du phosphate sont mesurées par le test de marquage au phosphate radioactif suivant :
La protéine est fixée sur une membrane de nitrocellulose (par exemple dot blot aspiration). La membrane est alors incubée avec un tampon Tris 50 mM pH 8,0 contenant du phosphate radioactif (³²P, 10mCi/mL, Amersham-Biosciences) 2M. La membrane est alors lavée deux fois avec le tampon ne comprenant plus de phosphate radioactif, et la membrane ainsi lavée est mise en contact avec un film auto-radiographique pendant environ 45 min. Le film est ensuite révélé et les impressions du film permettent de déterminer la capacité de HPBP à fixer le phosphate.

Selon un autre mode de réalisation, dans l'utilisation décrite ci-dessus, lesdites pathologies sont causées par les virus dont la transcription des gènes nécessite le facteur de transcription C/EBPβ, et notamment les virus VIH, les virus de l'Hépatite ou les virus de l'Herpes.

La transcription des gènes viraux est nécessaire à leur multiplication. Le génome des virus contient des gènes essentiels pour la synthèse des protéines spécifiques du virus (protéines d'enveloppe, enzymes...), et notamment des protéines possédant des propriétés transactivatrices puissantes telles que Tat pour le VIH, ICP27 pour le virus VHS, ou la protéine X de VHB.

Dans l'invention, on désigne par « virus de l'Hépatite » les virus induisant toute inflammation aiguë ou chronique du foie. L'art antérieur décrit 7 virus responsables de l'Hépatite : les virus VHA, VHB, VHC, VHD, VHE, VHF et VHD.

Un autre mode de réalisation avantageux de l'invention, décrit l'utilisation d'une composition telle que définie précédemment, dans laquelle lesdits virus du VIH sont les virus VIH-1 ou VIH-2.

Dans l'invention les virus VIH 1 sont représentés par leurs groupes M, O ou N, et également les sous groupes ou classes du groupe M : A, B, C, D, F, G, H, J et K.

Les souches virales mutantes, du fait de la faible fidélité de la réverse transcriptase, ainsi que les souches mixtes (issues d'une cellule infectée par deux souches virales distinctes) sont couvertes par l'invention.

Les souches virales mutantes peuvent également conduire à la résistance aux traitements communément utilisés ; il s'agit des souches dites résistantes.

Un mode de réalisation avantageux de l'invention décrit l'utilisation telle que définie ci-dessus, dans laquelle ladite composition est administrée à une dose d'environ 0,1 nM à environ 1mM, avantageusement d'environ 10nM à environ 100 nM, notamment d'environ 30nM à environ 70 nM, préférentiellement d'environ 50 nM.

La protéine HPBP possède une masse de 38 000 Da (38 kDa). Aussi, pour un individu adulte possédant en moyenne un volume sanguin de 5 L, la composition selon l'invention peut être administrée à une dose d'environ 0,3 µg/kg à environ 1mg/kg, notamment d'environ 30µg/kg à environ 300µg/kg, préférentiellement d'environ 90µg/kg à environ 210µg/kg, plus préférentiellement d'environ 150µg/kg.

La composition de l'invention peut être administrée notamment par voie intraveineuse, par voie sous-cutanée, par voie systémique, par voie locale au moyen d'infiltrations ou per os. Le traitement peut être continu ou séquentiel, c'est-à-dire au moyen d'une perfusion délivrant ladite composition de manière continue et éventuellement constante, ou sous forme discontinue, par une ou plusieurs prises ou injections quotidiennes, éventuellement renouvelées plusieurs jours, soit consécutifs, soit avec une latence sans traitement entre les prises.

Avantageusement, l'invention décrit l'utilisation d'une composition telle que défini précédemment, en association avec au moins un composé possédant des propriétés antivirales et/ou avec au moins un composé possédant des propriétés anti inflammatoires.

Par « composé possédant des propriétés antivirales » il est défini dans l'invention tout composé, protéique ou issus de la synthèse chimique, susceptible ou capable d'inhiber partiellement ou complètement au moins un des évènements nécessaire au cycle vital des virus.

Par cycle vital des virus, on défini dans l'inventions toutes les étapes de la vie d'un virus, de l'infection de la cellule hôte jusqu'à la production de nouveaux virus.

Comme exemple non limitatif, un composé possédant des propriétés antivirales selon l'invention peut inhiber dans le cadre du VIH : les processus de fusion cellulaire et d'entrée du virus, les processus de transcription inverse, les processus d'intégration dans le génome de la cellule hôte du génome du virus, les processus de transcription des gènes viraux, les processus de formation des virions ou encore les processus de maturation des différents constituants des virions néoformés.

Par « composé possédant des propriétés anti inflammatoire » il est défini dans l'invention tout composé, naturel ou issus de la synthèse chimique, susceptible ou capable d'inhiber partiellement ou complètement au moins un des évènements nécessaire à la mise en place d'une réponse inflammatoire telle que définie précédemment.

Un autre mode de réalisation préféré de l'invention décrit l'utilisation précédemment mentionnée, dans laquelle ledit composé antiviral possédant des propriétés antivirales est choisi parmi les inhibiteurs d'antiprotéases, notamment ritonavir, indinavir, saquinavir et nelfinavir, les inhibiteurs de transcriptase inverse, notamment l'AZT, ddI, ddC, 3TC, d4T, névirapine, delavirdine et efavirenz, les inhibiteurs de fusion, les inhibiteurs d'entrée et les inhibiteurs d'intégrase.

La composition selon l'invention peut également être utilisée en association avec les compositions antivirales indiquées dans le Tableau 1 suivant :

**Tableau 1 : Noms commerciaux des différents antiviraux utilisés pour le traitement du VIH, ainsi que leur spécificité d'action.**

| | |
|---|---|
| **Intelence®** (TMC 125/étravirine) Tibotec - | Inhibiteur non nucléosidique de la transcriptase inverse |
| **Agénérase**®(APV/amprénavir) GSK - | Inhibiteur de la protéase |
| **Aptivus**®(TPV/tipranavir) Boehringer - | Inhibiteur de la protéase |
| **Crixivan**®(IDV/indinavir) MSD - | Inhibiteur de la protéase |
| **Invirase**®(SQV/saquinavir) Roche - | Inhibiteur de la protéase |
| **Kalétra**®(LPV.r/lopinavir + ritonavir) Abbott | Inhibiteur de la protéase |
| **Norvir**®(ritonavir) Abbott - | Inhibiteur de la protéase |
| **Prezista**®(TMC 114/darunavir) Tibotec/Janssen-Cilag - | Inhibiteur de la protéase |
| **Reyataz**®(ATZ/atazanavir) BMS - | Inhibiteur de la protéase |
| **Telzir**®(APV/fosamprénavir) GSK - | Inhibiteur de la protéase |
| **Viracept**®(nelfinavir) Roche - | Inhibiteur de la protéase |
| **Fuzéon**®(T20/enfuvirtide) Roche - | Inhibiteur de fusion |
| **Celsentri**®(maraviroc) Pfizer - | Inhibiteur d'entrée |
| **Isentress**®(MK 0518/raltegravir) Merk - | Inhibiteur d'intégrase |
| **Rescriptor**®(delavirdine) Agouron - | Inhibiteur non nucléosidique de la transcriptase inverse |
| **Sustiva**®(EFV/efavirenz) BMS - | Inhibiteur non nucléosidique de la transcriptase inverse |
| **Viramune**®(névirapine) Boehringer - | Inhibiteur non nucléosidique de la transcriptase inverse |
| **Combivir**®(Rétrovir®+Epivir®) GSK - | Inhibiteur nucléosidique de la transcriptase inverse |
| **Emtriva**®(FTC, emtricitabine) Gilead - | Inhibiteur nucléosidique de la transcriptase inverse |
| **Epivir**®**(**3TC, lamivudine) GSK - | Inhibiteur nucléosidique de la transcriptase inverse |
| **Kivexa**®(Ziagen®+ Epivir®) GSK - | Inhibiteur nucléosidique de la transcriptase inverse |
| **Rétrovir**®(AZT/zidovudine) QSK - | Inhibiteur nucléosidique de la transcriptase inverse |
| **Trizivir**®(Rétrovir® + Epivir® + Ziagen®) GSK - | Inhibiteur nucléosidique de la transcriptase inverse |
| **Videx**®(ddI/didanosine) BMS - | Inhibiteur nucléosidique de la transcriptase inverse |
| **Viréad**®(TDF/ténofovir) Gilead - | Inhibiteur nucléosidique de la transcriptase inverse |
| **Zerit**®(d4T/stavudine) BMS - | Inhibiteur nucléosidique de la transcriptase inverse |
| **Ziagen**®(ABC/abacavir) GSK - | Inhibiteur nucléosidique de la transcriptase inverse |
| **Truvada**®(Emtriva® + Viréad®) Gilead - | Inhibiteur nucléosidique de la transcriptase inverse |
| **Atripla**®(Sustiva® + Emtriva® + Viréad®) BMS / GILEAD | Inhibiteur nucléosidique et non nucléosidique de la transcriptase inverse |

Un autre mode de réalisation préféré de l'invention décrit l'utilisation précédemment mentionnée, dans laquelle ledit composé possédant des propriétés anti-inflammatoires est choisi parmi les glucocorticoïdes de synthèse: à effets courts (prednisone), à effets intermédiaires (paraméthasone), à effets prolongés (bétaméthasone), ou les anti inflammatoires non stéroïdiens tels que les Salicylés (l'acide acétylsalicylique, le Salicylate de méthyle ou le diflunisal), les acides arylalkanoïques (l'Indométacine, le sulindac, ou le diclofénac), les Acides 2-arylpropioniques (profènes) (l'Ibuprofène, le Kétoprofène, le naproxène, ou le kétorolac), les acides N-arylanthraniliques (acides phénamiques) (l'acide méfénamique), les Oxicams (le piroxicam ou le méloxicam), les Coxibs (le célécoxib, le rofécoxib, le valdécoxib, le Parécoxib ou l'étoricoxib) ou encore les sulfonanilides (Nimésulide).

Egalement, un autre mode de réalisation avantageux de l'invention décrit l'utilisation d'une composition telle que définie précédemment, pour la préparation d'un médicament destiné à la prévention ou au traitement d' une infection virale, la dite composition et ledit composé antiviral possédant des propriétés antivirales ou ledit composé antiviral possédant des propriétés anti inflammatoire étant utilisés de manière simultanée, séparée ou étalée dans le temps.

Dans l'invention, la composition précédente est en association avec un composé antiviral possédant des propriétés antivirales ou anti-inflammatoires dans un rapport de environ 10/1 à environ 1/10, préférentiellement d'environ 5/1 à environ 1/5, plus particulièrement d'environ 2/1 à environ 1/2, et notamment 1/1.

L'invention décrit également une composition comprenant ou constituée par au moins un élément choisi parmi :
- une protéine déterminée comprenant ou constituée par la séquence d'acides aminés SEQ ID NO: 5,
- une protéine homologue de ladite protéine déterminée, possédant une séquence d'acides aminés présentant une homologie d'au moins 72% avec la séquence SEQ ID NO : 5,
sous réserve que lesdites protéines précédentes interagissent avec le facteur de transcription C/EBPβ et / ou la protéine virale Tat, pour la préparation d'un médicament destiné à la prévention ou au traitement d'une infection virale.

L'invention décrit également un produit de combinaison comprenant au moins un élément tel que défini précédemment et au moins un composé possédant des propriétés antivirales et/ ou des propriétés anti inflammatoires.

L'invention décrit également un produit de combinaison comprenant au moins un élément tel que défini précédemment et au moins un composé possédant des propriétés antivirales.

Un mode de réalisation avantageux de l'invention décrit un produit de combinaison susmentionné, pour la prévention ou au traitement de pathologies associées à une infection virale ou à une inflammation, la dite composition et ledit composé antiviral possédant des propriétés antivirales étant notamment utilisés de manière simultanée, séparée ou étalée dans le temps.

Un mode de réalisation avantageux de l'invention décrit un produit de combinaison susmentionné, pour la prévention ou au traitement de pathologies associées à une infection virale, la dite composition et ledit composé antiviral possédant des propriétés antivirales étant notamment utilisés de manière simultanée, séparée ou étalée dans le temps.

L'invention décrit également une composition pharmaceutique comprenant à titre de substance active au moins un élément choisi parmi
- une protéine déterminée comprenant ou constituée par la séquence d'acides aminés SEQ ID NO: 5, et
- une protéine homologue de ladite protéine déterminée, possédant une séquence d'acides aminés présentant une homologie d'au moins 72% avec la séquence SEQ ID NO:5,
sous réserve que lesdites protéines précédentes interagissent avec le facteur de transcription C/EBPβ et / ou la protéine virale Tat,
et au moins un composé antiviral possédant des propriétés antivirales, et/ou un composé possédant des propriétés anti inflammatoire
en association avec un véhicule pharmaceutiquement acceptable.

L'invention décrit également une composition pharmaceutique comprenant à titre de substance active
* au moins un élément choisi parmi
- une protéine déterminée comprenant ou constituée par la séquence d'acides aminés SEQ ID NO: 5, et
- une protéine homologue de ladite protéine déterminée, possédant une séquence d'acides aminés présentant une homologie d'au moins 72% avec la séquence SEQ ID NO : 5, sous réserve que lesdites protéines précédentes interagissent avec le facteur de transcription C/EBPβ et / ou la protéine virale Tat,
et
* au moins un composé antiviral possédant des propriétés antivirales,
en association avec un véhicule pharmaceutiquement acceptable.

Dans un mode de réalisation préféré, l'invention décrit une composition pharmaceutique telle que définie précédemment, où ladite protéine déterminée est constituée par la séquence d'acides aminés choisie parmi les séquences d'acides aminés suivantes : SEQ ID NO : 5, SEQ ID NO : 7 et SEQ ID NO : 9.

Un autre mode de réalisation préféré de l'invention décrit une composition pharmaceutique telle que définie précédemment, comprenant à titre de substance active au moins un élément choisi parmi
- une protéine déterminée comprenant ou constituée par la séquence d'acides aminés SEQ ID NO : 6, et
- une protéine homologue de ladite protéine déterminée, possédant une séquence d'acides aminés présentant une homologie d'au moins 72% avec la séquence SEQ ID NO : 6, et
sous réserve que lesdites protéines précédentes interagissent avec le facteur de transcription C/EBPβ et / ou la protéine virale Tat,
et au moins un composé possédant des propriétés antivirales et/ ou des propriétés anti inflammatoires,
en association avec un véhicule pharmaceutiquement acceptable.

Un autre mode de réalisation préféré de l'invention décrit une composition pharmaceutique telle que définie précédemment, comprenant à titre de substance active
* au moins un élément choisi parmi
- une protéine déterminée comprenant ou constituée par la séquence d'acides aminés SEQ ID NO : 6, et
- une protéine homologue de ladite protéine déterminée, possédant une séquence d'acides aminés présentant une homologie d'au moins 72% avec la séquence SEQ ID NO : 6, et
sous réserve que lesdites protéines précédentes interagissent avec le facteur de transcription C/EBPβ et / ou la protéine virale Tat,
et
* au moins un composé possédant des propriétés antivirales,
en association avec un véhicule pharmaceutiquement acceptable.

Dans un mode de réalisation préféré, l'invention décrit une composition pharmaceutique telle que définie précédemment, où ladite protéine déterminée est constituée par la séquence d'acides aminés choisie parmi les séquences d'acides aminés suivantes : SEQ ID NO : 6, SEQ ID NO : 8 et SEQ ID NO : 10.

Un autre mode de réalisation préféré de l'invention décrit une composition pharmaceutique telle que définie précédemment, comprenant à titre de substance active au moins un élément choisi parmi
- une protéine déterminée comprenant ou constituée par la séquence d'acides aminés SEQ ID NO : 1, et
- une protéine homologue de ladite protéine déterminée, possédant une séquence d'acides aminés présentant une homologie d'au moins 72% avec la séquence SEQ ID NO : 1, et
sous réserve que lesdites protéines précédentes interagissent avec le facteur de transcription C/EBPβ et / ou la protéine virale Tat,
et au moins un composé possédant des propriétés antivirales et/ ou des propriétés anti inflammatoires,
en association avec un véhicule pharmaceutiquement acceptable.

Un autre mode de réalisation préféré de l'invention décrit une composition pharmaceutique telle que définie précédemment, comprenant à titre de substance active
* au moins un élément choisi parmi
- une protéine déterminée comprenant ou constituée par la séquence d'acides aminés SEQ ID NO : 1, et
- une protéine homologue de ladite protéine déterminée, possédant une séquence d'acides aminés présentant une homologie d'au moins 72% avec la séquence SEQ ID NO : 1, et
sous réserve que lesdites protéines précédentes interagissent avec le facteur de transcription C/EBPβ et / ou la protéine virale Tat,
et
* au moins un composé possédant des propriétés antivirales,
en association avec un véhicule pharmaceutiquement acceptable.

Dans un mode de réalisation préféré, l'invention décrit une composition pharmaceutique telle que définie précédemment, où ladite protéine déterminée est constituée par la séquence d'acides aminés choisie parmi les séquences d'acides aminés suivantes : SEQ ID NO : 1, SEQ ID NO : 2 et SEQ ID NO : 3.

Dans l'invention, la composition pharmaceutique précédente est en association avec un composé possédant des propriétés antivirales dans un rapport de environ 10/1 à environ 1/10, préférentiellement d'environ 5/1 à environ 1/5, plus particulièrement d'environ 2/1 à environ 1/2, et notamment 1/1.

Pour un individu adulte possédant en moyenne un volume sanguin de 5 L, la composition selon l'invention peut être administrée à une dose d'environ 0,3 µg/kg à environ 1mg/kg, notamment d'environ 30µg/kg à environ 300µg/kg, préférentiellement d'environ 90µg/kg à environ 210µg/kg, plus préférentiellement d'environ 150µg/kg.

Dans l'invention, la composition pharmaceutique précédente est en association avec un composé possédant des propriétés anti-inflammatoires dans un rapport de environ 10/1 à environ 1/10, préférentiellement d'environ 5/1 à environ 1/5, plus particulièrement d'environ 2/1 à environ 1/2, et notamment 1/1.

Pour un individu adulte possédant en moyenne un volume sanguin de 5 L, la composition selon l'invention peut être administrée à une dose d'environ 0,3 µg/kg à environ 1mg/kg, notamment d'environ 30µg/kg à environ 300µg/kg, préférentiellement d'environ 90µg/kg à environ 210µg/kg, plus préférentiellement d'environ 150µg/kg.

Dans l'invention, la composition pharmaceutique précédente est en association avec un composé possédant des propriétés antivirales et un composé possédant des propriétés anti-inflammatoires dans un rapport de environ 10/1/1 à environ 1/10/10, préférentiellement d'environ 5/1/1 à environ 1/5/5, plus particulièrement d'environ 2/1/1 à environ 1/2/2, et notamment 1/1/1.

Pour un individu adulte possédant en moyenne un volume sanguin de 5 L, la composition selon l'invention peut être administrée à une dose d'environ 0,3 µg/kg à environ 1mg/kg, notamment d'environ 30µg/kg à environ 300µg/kg, préférentiellement d'environ 90µg/kg à environ 210µg/kg, plus préférentiellement d'environ 150µg/kg.

La composition pharmaceutique selon l'invention peut éventuellement être une composition neutracétique ou cosmétique.

Un autre mode de réalisation avantageux de l'invention décrit une composition pharmaceutique telle que définie ci-dessus, dans laquelle lesdites pathologies sont causées par une infection par les virus du HIV, les virus de l'Hépatite ou les virus de l'Herpes.

Dans un autre mode de réalisation avantageux, l'invention décrit une composition pharmaceutique précédemment décrite, dans laquelle lesdits virus du VIH sont les virus VIH1 ou VIH2.

Un autre mode de réalisation avantageux de l'invention décrit une composition pharmaceutique définie précédemment, dans laquelle ledit composé antiviral est choisi parmi les inhibiteurs d'antiprotéases, notamment ritonavir, indinavir, saquinavir et nelfinavir, et les inhibiteurs de transcriptase inverse, notamment l'AZT, ddI, ddC, 3TC, d4T, névirapine, delavirdine et efavirenz.

Un autre mode de réalisation avantageux de l'invention décrit une composition pharmaceutique définie précédemment, dans laquelle ledit composé anti inflammatoire est choisi parmi les glucorticoïdes de synthèse, notamment la Prednisone, la Prednisolone, la Méthylprednisolone, la Dexaméthasone, la Bétaméthasone ou encore la Paraméthasone, les anti inflammatoires non stéroïdiens, notamment les Salicylés tels que le Salicylate de méthyle et le Diflunisal, les Acides arylalkanoïques tels que l'Indométacine, le Sulindac et le Diclofénac, les Acides 2-arylpropioniques (profènes) tels que l'Ibuprofène, le Kétoprofène, le Naproxène et le Kétorolac , les Acides N-arylanthraniliques (acides phénamiques) tel que l'Acide méfénamique, les Oxicams tels que le Piroxicam et le Méloxicam, les Coxibs tels que le Célécoxib, le Rofécoxib, leValdécoxib, le Parécoxib, ou l'Étoricoxib, et les Sulfonanilides tel que le Nimésulide.

Egalement, l'invention décrit une méthode de diagnostic *in vitro,* de la résistance à une infection par un virus, dans un échantillon biologique issu d'un individu, comprenant,
- la détermination dans ledit échantillon biologique de la quantité de protéine déterminée telle que définie précédemment,
- la comparaison de ladite quantité obtenue à l'étape suivante avec la quantité de protéine déterminée d'au moins un échantillon de référence,
- la détermination, à partir de la comparaison précédente, de la résistance à une infection virale.

Par « la résistance à une infection par un virus », il est défini dans l'invention la capacité qu'ont certains individus de ne pas être infectés par des virus. En effet, dans les conditions normales, un individu non résistant, lorsqu'il est mis en présence d'un virus pour la première fois (primo-infection), est normalement infecté par ledit virus. Dans certains rares cas, certains individus sont résistants à la primo infection, c'est-à-dire que lors de la première mise en présence de l'individu avec le virus, celui-ci n'est pas capable d'infecter ledit individu.

Dans l'invention, on entend par « infecter », la capacité que possède un virus de détourner la machinerie cellulaire de la cellule hôte à son profit, c'est à dire produire de nouveaux virus ou virions. Le fait de « ne pas être capable d'infecter » signifie que le virus n'est pas capable d'achever efficacement la production de nouveaux virions.

Dans la méthode de l'invention, la quantité en protéine HPBP est déterminée dans un échantillon biologique tel que le sang, le plasma, la lymphe ou encore l'urine. « La détermination de la quantité » signifie que la protéine HPBP est dosée dans ledit échantillon. Les méthodes de dosage de la protéine HPBP peuvent être des méthodes immunologiques connues de l'homme de métier. Par exemple, les techniques de Western Blot (immunodétection), d'immunoprécipitation, ou les techniques d'ELISA peuvent être utilisées, également les techniques de RIA peuvent être mises en oeuvre.

Un exemple de détection de la protéine HPBP est l'ELISA décrit dans la partie Exemple. Succinctement, un anticorps monoclonal ou polyclonal dirigé contre la protéine HPBP est immobilisé sur une plaque. Après lavages, l'échantillon est incubé avec la plaque ou lesdits anticorps sont immobilisés. Après lavages, un second anticorps dirigé contre la protéine HPBP est incubé avec ladite plaque préalablement incubée avec l'échantillon. Enfin, après lavage, la plaque est incubée avec un anticorps dirigé contre la partie constante des anticorps précédents, lesdits anticorps dirigés contre la partie constante étant couplés à une molécule permettant la détection telle que la peroxidase (HRP).

Le dosage de la protéine HPBP ainsi réalisé peut être quantifié par des techniques photométriques ou spectroscopiques bien connues de l'homme de métier.

La concentration de la protéine HPBP est comparée à la concentration d'au moins un échantillon de référence. L'échantillon de référence peut correspondre :
- soit à un échantillon issu d'un individu sain,
- soit à un échantillon issu d'un un individu ayant été infecté par un virus, notamment un individu infecté par le VIH, ledit individu ayant développé ou non des symptômes liées à ladite infection virale, soit
- un échantillon issu d'un individu résistant à l'infection virale, ledit individu résistant ayant été en contact une ou plusieurs fois avec ledit virus sans jamais être infecté.

La méthode selon l'invention permet de déterminer si un individu est résistant à une infection virale.

Si le taux de HPBP est similaire, ou substantiellement le même que celui d'un individu sain ou d'un individu infecté par un virus, l'échantillon de l'individu est considéré comme étant susceptible d'être infecté par ledit virus : l'individu n'est pas résistant.

Si le taux de HPBP est élevé, ou similaire à celui d'un individu résistant à une infection virale, l'individu d'où provient l'échantillon testé sera considéré comme étant résistant à une infection virale.

L'invention concerne également une méthode de diagnostic définie précédemment, dans laquelle ladite infection est due à l'infection par les virus du VIH, notamment le virus VIH1 ou VIH2, les virus de l'Hépatite ou les virus de l'Herpes.

L'invention décrit également une composition pharmaceutique comprenant à titre de substance active au moins un élément choisi parmi
- une protéine déterminée caractérisée en ce qu'elle comprend ou est constituée d'une séquence d'acide aminée choisie parmi SEQ ID NO : 5, SEQ ID NO : 6 et SEQ ID NO :1, et
- une protéine homologue de ladite protéine déterminée, caractérisée en ce qu'elle possède une homologie d'au moins 72% avec l'une des séquences susmentionnées,
sous réserve que lesdites protéines précédentes interagissent avec le facteur de transcription C/EBPβ et / ou la protéine virale Tat,
et au moins un composé possédant des propriétés antivirales et/ou au moins un composé possédant des propriétés anti-inflammatoires,
ledit composé antiviral étant notamment choisi parmi les inhibiteurs d'antiprotéases, tels que ritonavir, indinavir, saquinavir et nelfinavir, et les inhibiteurs de transcriptase inverse, tels que l'AZT, ddI, ddC, 3TC, d4T, névirapine, delavirdine et efavirenz.,
en association avec un véhicule pharmaceutiquement acceptable,
pour la prévention ou le traitement de pathologies associées à une infection virale ou à une inflammation.

L'invention décrit également une composition pharmaceutique comprenant à titre de substance active
* au moins un élément choisi parmi
- une protéine déterminée caractérisée en ce qu'elle comprend ou est constituée d'une séquence d'acide aminée choisie parmi SEQ ID NO : 5, SEQ ID NO : 6 et SEQ ID NO :1, et
- une protéine homologue de ladite protéine déterminée, caractérisée en ce qu'elle possède une homologie d'au moins 72% avec l'une des séquence susmentionnée,
sous réserve que lesdites protéines précédentes interagissent avec le facteur de transcription C/EBPβ et / ou la protéine virale Tat,
et
* au moins un composé possédant des propriétés antivirales choisi notamment parmi les inhibiteurs d'antiprotéases, tels que ritonavir, indinavir, saquinavir et nelfinavir, et les inhibiteurs de transcriptase inverse, tels que l'AZT, ddI, ddC, 3TC, d4T, névirapine, delavirdine et efavirenz.,
en association avec un véhicule pharmaceutiquement acceptable,
pour la prévention ou le traitement d' infection virale.

L'invention décrit également dans un mode de réalisation avantageux une composition pharmaceutique telle que définie précédemment où ladite protéine déterminée comprend ou est constituée par la séquence d'acide aminé choisie parmi les séquences d'acides aminés suivantes : SEQ ID NO : 1, SEQ ID NO : 2, SEQ ID NO : 3, SEQ ID NO : 6, SEQ ID NO : 7, SEQ ID NO : 8, SEQ ID NO : 9 ou SEQ ID NO : 10.

La présente invention sera mieux illustrée par les exemples et les figures suivantes, ceux-ci n'ayant pas de caractère limitatif.

Les figures 1 à 12 illustrent l'invention.
La figure 1 représente la structure tridimensionnelle de la protéine HPBP.
La figure 2 représente un gel d'électrophorèse coloré au nitrate d'argent montrant dans la piste A la purification du complexe PON-1/HPBP, et dans les pistes B et C les produits de la séparation après passage sur colonne hydroxylapatide des protéines PON-1 et HPBP, respectivement. Les poids moléculaires sont indiqués.

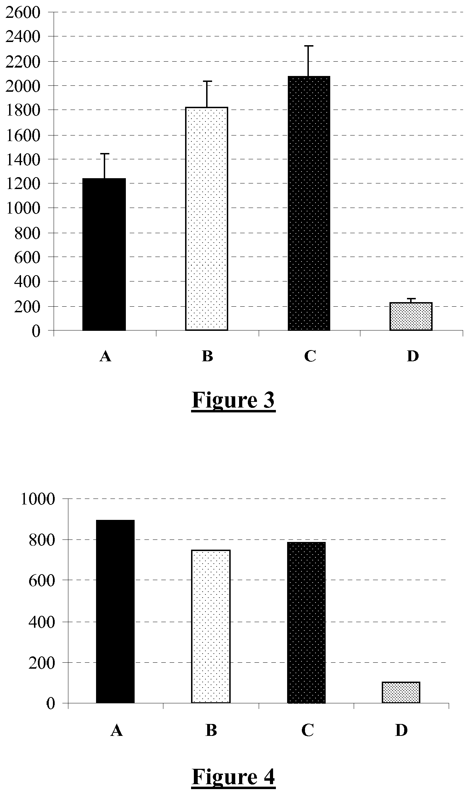

La figure 3 représente la mesure de l'activité transcriptionnelle du virus VIH dans des lignées Jurkat. Les valeurs représentent l'activité luciférase. La Piste A représente les cellules non traitées, la piste B représente les cellules traitées avec le mélange de protéines PON-1 et HPBP, la piste C représente les cellules traitées avec la protéine PON-1, la piste D représente les cellules traitées avec la protéine HPBP.
La figure 4 représente la mesure de l'activité de réplication du virus VIH dans des lignées Jurkat. Les valeurs représentent la quantité de protéine p24. La Piste A représente les cellules non traitées, la piste B représente les cellules traitées avec le mélange de protéines PON-1 et HPBP, la piste C représente les cellules traitées avec la protéine PON-1, la piste D représente les cellules traitées avec la protéine HPBP.

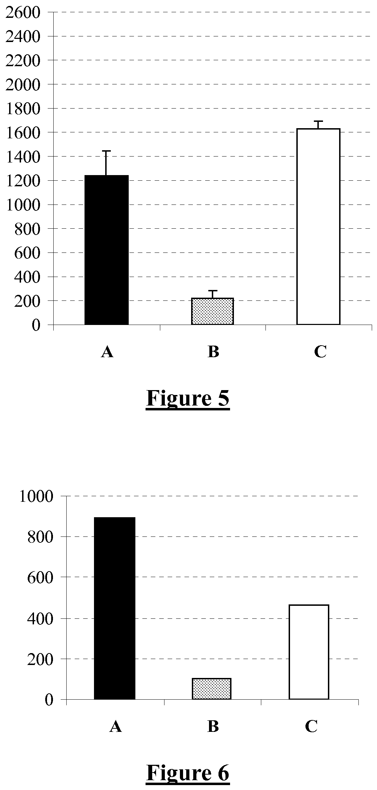

La figure 5 représente la mesure de l'activité transcriptionnelle du virus VIH dans des lignées Jurkat. Les valeurs représentent l'activité luciférase. La Piste A représente les cellules non traitées, la piste B représente les cellules traitées avec la protéine HPBP, la piste C représente les cellules traitées à l'AZT.
La figure 6 représente la mesure de l'activité de réplication du virus VIH dans des lignées Jurkat. Les valeurs représentent la quantité de protéine p24. La Piste A représente les cellules non traitées, la piste B représente les cellules traitées avec la protéine HPBP, la piste C représente les cellules traitées à l'AZT.

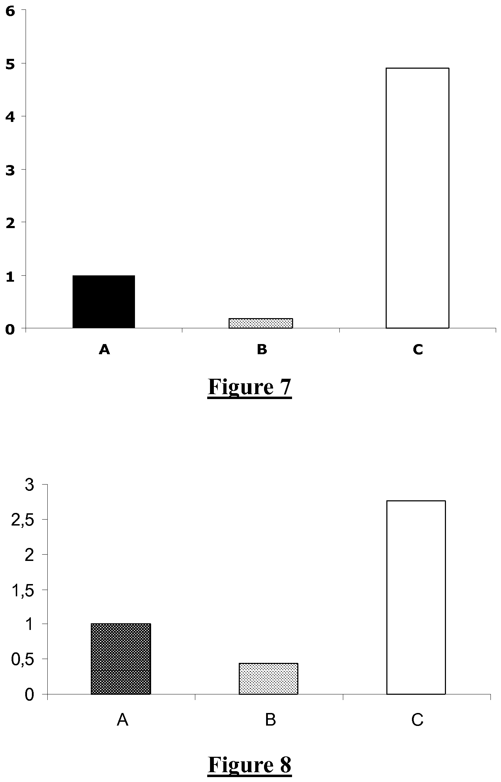

La figure 7 représente la mesure de l'activité transcriptionnelle du virus VIH dans des lignées Jurkat. Les valeurs représentent le taux d'induction.de la transcription. La Piste A représente les cellules non traitées, la piste B représente les cellules traitées avec HPBP, la piste C représente les cellules traitées avec la protéine DING de pomme de terre.
La figure 8 représente la mesure de l'activité de réplication du virus VIH dans des lignées Jurkat. Les valeurs représentent le taux d'induction.de la réplication La Piste A représente les cellules non traitées, la piste B représente les cellules traitées avec HPBP, la piste C représente les cellules traitées avec la protéine DING de pomme de terre.

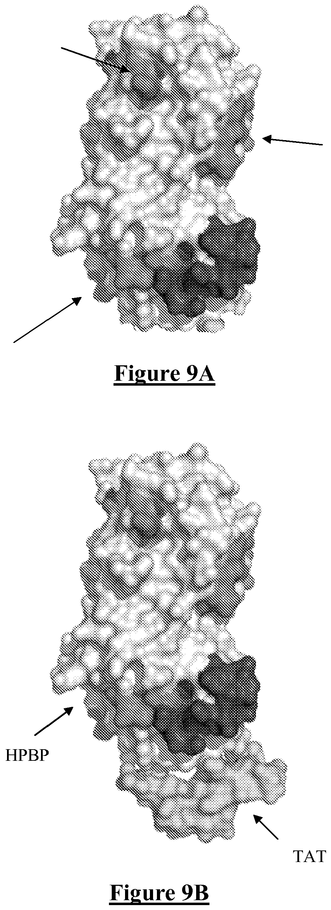

Les figures 9A et 9B représentent la modélisation moléculaire de l'interaction HPBP/Tat. La figure 9A représente la structure tridimensionnelle de la protéine HPBP où sont indiqués les épitopes reconnus par les anticorps. Les régions en gris foncé 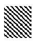 localisées par des flèches (→) représentent les épitopes n'interférant pas avec l'interaction HPBP/Tat, la région en noir 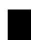 représente la zone d'interaction avec la protéine Tat. La figure 9B représente la modélisation moléculaire du complexe HPBP/Tat.

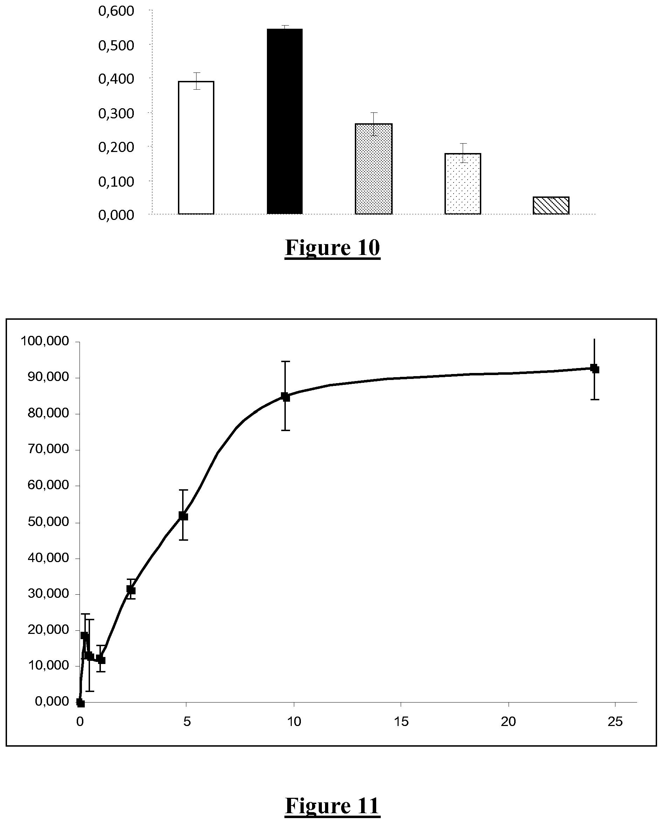

La figure 10 représente des histogrammes mesurant la toxicité cellulaire de la protéine HPBP. La barre blanche représente les cellules contrôle non traitées, la barre noire représente des cellules traitées avec de la protéine HPBP à une concentration de 0,2µg.mL⁻¹, la barre grise représente des cellules traitées avec de la protéine HPBP à une concentration de 2µg.mL⁻¹, la barre avec des points représente des cellules traitées avec de la protéine HPBP à une concentration de 20 µg.m⁻¹ et la barre hachurée représente des cellules traitées avec de la protéine HPBP à une concentration de 0,2µg.mL⁻¹. L'axe des abscisses représente la densité optique mesurée à 570 nm.
La figure 11 représente une courbe de toxicité de la protéine HPBP (exprimé en %), en fonction de la quantité de protéine. Cette courbe est obtenue à partir de cellules lymphocytaires issues de sang périphérique.

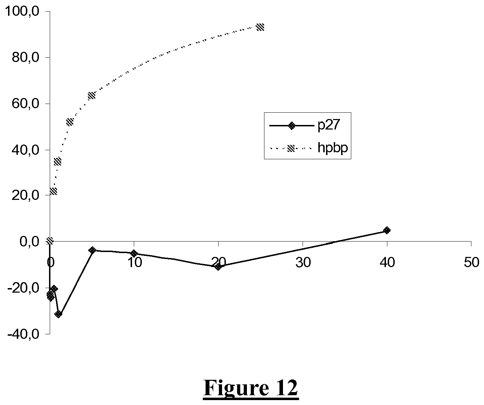

La figure 12 représente une courbe de toxicité (exprimée en %)de la protéine p27^{SJ} de millepertuis (trait plein) ou de la HPBP (trait pointillé), en fonction de la quantité de protéine. Cette courbe est obtenue à partir de cellules microgliales.

### PARTIE EXPERIMENTALE

Sauf indications contraires, toutes les expériences mentionnées ci-après ont été réalisées au moins 3 fois, de manières indépendantes.

### Exemple 1 : Purification de la protéine HPBP

### Etape 1 : purification du complexe HPBP/PON-1

La protéine SEQ ID NO : 2 est purifiée à partir de poches de plasma congelé (-200 mL) fournies par l'Etablissement de Transfusion Sanguine de Lyon-Beynost. Le caillot de fibrine, formé par l'ajout de 1 M (1% v/v) de CaCl₂ au plasma est séparé du sérum par filtration. Le sérum est alors mélangé à 400 mL de Gel d'affinité (Cibacron 3GA-Agarose, C-1535, Sigma) équilibré avec un tampon A (Tris/HCl 50 mM, CaCl₂ 1mM, NaCl 4M, pH 8). Dans ces conditions, principalement les HDL ("high density lipoprotein" : lipoprotéines de haute densité) sont adsorbées. Après 6 à 8 heures d'incubation, les protéines non adsorbées sur le gel sont éliminées par filtration sur fritté de porosité n°2. Ce lavage s'effectue jusqu'à ce que l'on ne détecte plus de protéine dans l'éluat (absorption UV à 280 nm). Le gel est ensuite équilibré avec un tampon B (Tris/HCl 50 mM, CaCl₂ 1mM, pH 8) puis placé en colonne XK 50/30 (Pharmacia). L'élution est réalisée en rajoutant 1g/l de déoxycholate de sodium et 0,1% de triton X-100 au tampon B. Les fractions montrant une activité arylestérase sont mélangées et injectées sur 50 mL d'un gel échangeur d'anions (DEAE Sepharose Fast Flow, Pharmacia) disposé en colonne XK 26/70 (Pharmacia) et équilibré avec le tampon B et 0,1% de triton X-100. L'élution se fait par gradient de NaCl. Un premier palier est réalisé à 87,5 mM de NaCl afin d'éliminer l'apo A-I, une protéine liée à la paraoxonase, et la majorité des protéines contaminantes. La paraoxonase humaine (PON1) est environ éluée à la concentration de 140 mM de NaCl.

La protéine HPBP copurifie avec la protéine PON-1.

### Etape 2 : Séparation des protéines PON-1 et HPBP

Les complexes PON-1/HPBP sont séparés sur colonne d'hydroxyapatite (systeme AKTA FPLC (Amersham Biosciences) équipé avec un logiciel UNICORN (version 3.2) pour contrôler les unités de séparation. La chromatographie est réalisée à 25 °C).

Les fractions de protéines actives issues de la colonne échangeuse d'ion sont chargées sur une colonne de verre (1 cm×10 cm, Amersham) contenant 2ml de résine hydroxyapatite Bio-Gel HTP à un débit de 0,5mL/min.

La résine hydroxyapatite est équilibrée au préalable avec un tampon phosphate de sodium 10mM à pH 7.0 avec ou sans 0.1% Triton (Tampon A).

Après lavage avec le tampon A, les protéines sont éluées de manière linéaire en augmentant la force ionique du tampon phosphate de sodium de 10 à 400 mM (pH 7,0), ou directement à 400 nM de tampon phosphate de sodium. L'Equilibration et l'élution sont réalisées sans calcium.

La protéine PON-1 est éluée à une concentration d'environ 0.3mg/mL, concentration dépendante de l'échantillon humain de départ.

La protéine HPBP est éluée à une concentration d'environ 0.05mg/mL, concentration dépendante de l'échantillon humain de départ.

Les fractions collectées sont analysées sur gel SDS-PAGE de concentration en polyaccrylamide de 10%. Les protéines sont séparées pendant 35 min à voltage constant (200V). Les protéines une fois séparées sont visualisées par coloration au nitrate d'argent (Silver Stain Plus kit, Bio-Rad).

Un gel de séparation des protéines isolées est représenté sur la figure 2.

### Exemple 2 : interaction in vitro des protéines HPBP et C/EBPβ

L'association de la Human Phosphate Binding Protein (HPBP) avec la protéine C/EBPβ est réalisée en utilisant une technique d'ELISA.

Dans des plaques ELISA 96-puits à fond plats ayant une haute affinité de liaison pour les protéines la protéine C/EBPβ est adsorbée sur le support et incubée une nuit à 4°C.

Les protéines non fixées sur la plaque sont éliminées par lavage avec un tampon Phosphate Buffered Saline (PBS) contenant 10mg/mL de Bovine Serum Albumine (BSA).

Les puits sont saturés par une solution tampon Phosphate Buffered Saline (PBS) contenant 20mg/mL de Bovine Serum Albumine (BSA) pendant 1h à température ambiante afin d'éliminer le bruit de fond.

Après lavage grâce à une solution de Phosphate Buffered Saline (PBS) contenant 10mg/mL de Bovine Serum Albumine (BSA), différentes solutions de HPBP pure de 0.1nM à 100mM sont ajoutées dans les puits pour une incubation 1-3h à RT.

La plaque est une nouvelle fois lavée puis incubée avec des anticorps anti-HPBP monoclonaux de souris ou des anticorps polyclonaux anti-HPBP de lapin pour une incubation de 1-3h à RT.

Finalement, après un nouveau lavage, des anticorps couplés à la Horseradish Peroxydase (HRP) reconnaissant les fragments Fc de souris ou de lapin sont ajoutées dans les puits pour une incubation 1-3h à RT.

Puis après un dernier lavage, la plaque est incubée avec un substrat de la HRP (la 3,3',5,5'-tétraméthylbenzidine : TNB en présence d'eau oxygénée H₂O₂). La réaction est arrêtée après 10 min par ajout de H₂SO₄. La densité optique du produit de la réaction est alors mesurée avec un spectrophotomètre à 450 nm.

Les résultats montrent que la protéine HPBP et la protéine C/EBPβ sont capables d'interagir in vitro.

### Exemple 3 : Interaction in vivo des protéines HPBP et C/EBPβ ou HPBP et Tat

Afin de valider l'interaction entre HPBP et C/EBPβ, des expériences de co-immunoprécipitation ont été réalisées.

Des lignées cellulaires humaines transformées (Cellules Jurkat, Cellules HeLa...) ou des cellules primaires (lymphocytes circulants...) sont incubées avec une solution de protéine HPBP.

Les cellules sont alors lysées et la protéine HPBP est immunoprécipitée à l'aide d'anticorps spécifiques, dans un tampon permettant de préserver les interactions protéiques.

Les protéines immunoprécipitées sont séparées sur gel selon la technique SDS-PAGE, les protéines sont transférées sur membrane de nitrocellulose ou PVDF, et la présence de la protéine C/EBPβ est révélée par la technique de Wester-blot à l'aide d'anticorps spécifiques dirigés contre la protéine C/EBPβ.

Des expériences similaires sont réalisées en immunoprécipitant la protéine C/EBPβ et en révélant la présence de la protéine HPBP.

Afin de valider l'interaction entre HPBP et Tat, des expériences de co-immunoprécipitation ont été réalisées.

Des lignées cellulaires humaines transformées (Cellules Jurkat, lignées macrophagiques...) ou des cellules primaires (lymphocytes circulants...) infectées par le VIH, ou exprimant la protéine Tat recombinante, sont incubées avec une solution de protéine HPBP.

Les cellules sont alors lysées et la protéine HPBP est immunoprécipitée à l'aide d'anticorps spécifiques, dans un tampon permettant de préserver les interactions protéiques.

Les protéines immunoprécipitées sont séparées sur gel selon la technique SDS-PAGE, les protéines sont transférées sur membrane de nitrocellulose ou PVDF, et la présence de la protéine Tat est révélée par la technique de Western-blot à l'aide d'anticorps spécifiques dirigés contre la protéine Tat.

Des expériences similaires sont réalisées en immunoprécipitant la protéine Tat et en révélant la présence de la protéine HPBP.

### Exemple 4 : HPBP interfère avec l'interaction C/EBPβ-Tat

L'interaction C/EBPβ-Tat de HIV a été décrite par [Abraham et al. J Neuroimmunol. 2005 Mar;160(1-2):219-27].

Afin de mesurer l'interférence de la protéine HPBP sur l'interaction C/EBPβ-Tat, un test ELISA a été réalisé.

Dans des plaques ELISA 96-puits à fond plats ayant une haute affinité de liaison pour les protéines la protéine C/EBPβ est adsorbée sur le support et incubée une nuit à 4°C.

Les protéines non fixées sur la plaque sont éliminées par lavage avec un tampon Phosphate Buffered Saline (PBS) contenant 10mg/mL de Bovine Serum Albumine (BSA).

Les puits sont saturés par une solution tampon Phosphate Buffered Saline (PBS) contenant 20mg/mL de Bovine Serum Albumine (BSA) pendant 1h à température ambiante afin d'éliminer le bruit de fond.

Après lavage grâce à une solution de Phosphate Buffered Saline (PBS), différentes solutions de HPBP pure de 0.1nM à 100mM sont ajoutées dans les puits pour une incubation 1-3h à RT.

La plaque est une nouvelle fois lavée puis incubée avec de la protéine TAT à differentes concentration pendant 1-3h à température ambiante.

La plaque est une nouvelle fois lavée puis incubée avec des anticorps anti-TAT monoclonaux de souris ou des anticorps polyclonaux anti-TAT de lapin pour une incubation de 1-3h à RT. Finalement, après un nouveau lavage, des anticorps couplés à la Horseradish Peroxydase (HRP) reconnaissant les fragments Fc de souris ou de lapin sont ajoutées dans les puits pour une incubation 1-3h à RT.

Puis après un dernier lavage, la plaque est incubée avec un substrat de la HRP (la 3,3',5,5'-tétraméthylbenzidine : TNB en présence d'eau oxygénée H₂O₂). La réaction est arrêtée après 10 min par ajout de H₂SO₄. La densité optique du produit de la réaction est alors mesurée avec un spectrophotomètre à 450 nm.

### Exemple 6 : HPBP inhibe la réplication et inhibe la transcription du génome du virus VIH

Méthode : une lignée cellulaire de Lymphocyte T CD4+ Jurkat (lignée 1G5) ayant intégré dans son génome une construction comprenant un gène rapporteur luciférase dont l'expression est contrôlée par le promoteur du VIH-1 (LTR du VIH-1; lignée Jurkat LTR-HIV) a été utilisée. La mesure d'une activité luciférase à partir des extraits cellulaires permet donc d'estimer l'activité transcriptionnelle du VIH-1.

Les lignées Jurkat LTR-HIV et Jurkat 1G5 ont été transfectées grâce à la technique du DEAE-dextran avec le génome proviral NL4.3 (génome VIH-1 complet, souche LAI).

Les lignées Jurkat LTR-HIV et Jurkat 1G5 ainsi infectées, sont traités 24 h plus tard avec les complexes de protéines HPBP/PON ou avec les protéines purifiées PON ou HPBP à une concentration finale de 2 µg/mL de milieu (52nM de protéine seule).

Les cellules et le surnageant cellulaire sont prélevés après 24h de traitement.

L'activité transcriptionnelle est mesurée par la réalisation d'un essai luciférase. L'intensité de la réplication du VIH-1 est mesurée grâce au dosage ELISA de la quantité de protéines de capsides virales p24 présentes dans le surnageant des cultures cellulaires des lignées 1G5 infectées [Rohr, O., et al. (2000). The Journal of biological chemistry 275, 2654-2660].

Un contrôle négatif non traité ainsi qu'un contrôle positif traité avec de l'AZT à une concentration finale de 10µM ont été réalisés en parallèle. Chaque expérience est réalisée en triplicata.

En parallèle, les lignées lymphocytaires infectées obtenus ont été traitées avec 10µg de la protéine DING de pomme de terre.

Les résultats montrent HPBP exerce un effet répresseur sur la réplication du VIH-1 (Figure 4) dans les lymphocytes TCD4+ (89% d'inhibition). Par contre, PON-1 et HPBP, ou la n'exerce pas d'effet répresseur. De plus, un mélange PON-1/HPBP purifié selon un protocole standard ne présente pas de propriétés d'inhibition aussi importantes que la protéine HPBP seule.

L'inhibition de la réplication semble passer par une inhibition de l'activité transcriptionnelle qui est réprimée à 80% par HPBP (Figure 3). De la même manière que pour l'activité réplicative, un mélange des protéines PON-1 et HPBP, ou la protéine PON-1 seule n'exerce un tel effet inhibiteur.

L'inhibition de la réplication du VIH par la protéine HPBP est significativement plus importante (89% d'inhibition) que celle observée par le traitement avec 10µM d'AZT (60% d'inhibition (Figure 6).

De plus, HPBP inhibe la réplication virale de plus d'un facteur 6, alors que l'AZT n'a pas d'effet sur la réplication du virus (Figure 5).

L'activité anti-VIH de HPBP semble passer par une inhibition de l'étape de transcription du virus. Cette étape du cycle viral n'est pas encore ciblée par les stratégies thérapeutiques actuelles. Les souches de virus résistantes aux molécules utilisées dans le cadre d'une multi thérapie active (HAART) n'ont donc pas subi de pression de sélection permettant de prévoir une quelconque résistance au traitement par HPBP. L'efficacité répressive observée pour la souche HIV-1 NL4.3 devrait donc logiquement être comparable pour d'autres souches de virus dont les souches résistantes aux thérapies.

Les expériences avec la protéine DING de pomme de terre montrent que de manière inattendue, la protéine homologue à HPBP n'est pas capable d'inhiber la réplication du VIH, ni même d'inhiber la réplication du VIH. Au contraire la protéine de pomme de terre exerce une activité stimulatrice de la réplication de plus de 3 fois (figure 6) et une activité stimulatrice de la transcription de 5 fois (figure 5).

### Exemple 7 : HPBP inhibe l'expression des gènes proinflammatoires MCP-1 et IL-6.

Des lignées cellulaires transformées avec des constructions où le gène de la luciférase est mis sous contrôle des séquences régulatrices du gène MCP-1 ou du gène IL-6 sont traitées ou non avec un inducteur de l'inflammation (LPS), et traitées ou non en parallèle avec de la protéine HPBP.

L'induction de la luciférase est révélée à l'aide du substrat ad hoc (par exemple luciférine), et l'activité luciférase est mesurée à l'aide d'un luminomètre.

### Exemple 8 : Dose reponse de l'inhibition du VIH par HPBP.

La concentration de protéine HPBP (concentration inhibitrice 50 ; CI₅₀) la plus efficace pour inhiber la réplication et la transcription du VIH-1 a été évaluée selon la méthode décrite dans l'Exemple 6.

Les cellules ont été traitées avec des gammes de concentration de protéine HPBP variant de 0.1nM à 100mM.

### Exemple 9 : Mesure de la toxicité des protéines in vivo :

La toxicité de la protéine HPBP est évaluée en mesurant la prolifération et la viabilité de lignées cellulaires humaines Jurkat traitées avec des doses de HPBP variant de 0,1nM à 100mM.

La prolifération est mesurée par comptage des cellules quotidiennement, après coloration au bleu de Trypan afin d'exclure les cellules mortes, ou par marquage au MTT (cf. infra).

Une méthode alternative consiste en la mesure de l'incorporation de thymidine tritiée (³H) qui témoigne de la réplication de l'ADN, et donc de la division cellulaire.

La viabilité de ces mêmes cellules peut être estimée par différentes techniques, notamment le dénombrement de cellules positives au bleu de Trypan (cellules ayant incorporé le colorant). D'autres techniques de mesure de l'apoptose sont également utilisées telles que la mesure de la quantité d'ADN fragmenté dit « subG1 » par cytométrie de flux, ou la mesure de marqueur précoces (Anexin V) ou tardifs (fragmentation nucléosomale de l'ADN) de l'apoptose.

### Exemple 10 : Inhibition des souches de VIH résistantes aux thérapies classiques

En France en 2003-2004, la fréquence de virus résistant à au moins un antirétroviral chez les patients diagnostiqués au moment de leur primo-infection était de 12.3 %. La fréquence de virus résistant aux INTIs est de 6 %, aux INNTIs de 5.9 % et aux IPs de 3.4 %.

Les mécanismes de résistance et les mutations impliquées dans la résistance aux antirétroviraux sont essentiellement connus pour les isolats VIH-1 sous-type B (6), qui circulent majoritairement dans les pays développés.

L'inhibition par la protéine HPBP de la réplication virale et de la transcription virale d'une souche résistante à l'AZT (génome proviral NL4.3 (génome VIH-1 complet, souche LAI) possédant la mutation T215Y a été testée, selon le protocole décrit dans l'exemple 6.

### Exemple 11 : Les tests de l'action de HPBP sur les autres cellules infectées par le VIH.

Le VIH est capable d'infecter, outre les cellules lymphocytaires T, les cellules macrophagiques, dendritiques et microgliales.

L'inhibition de la réplication virale et de la transcription virale est évaluée comme décrit dans l'Exemple 6, en utilisant les cellules suivantes :
- cellules macrophagiques humaines THP-1 (ATCC n° TIB-202™), et
- cellules microgliales humaines : HMO6 [Nagai A. et al. (2001)Neurobiology of Disease 8, 1057-1068].

### Exemple 12 : Dosage de la HPBP dans des cohortes de patients non progressifs

La concentration en protéine plasmatique HPBP a été testée chez des patients non progressifs grâce à un ELISA HPBP.

Dans des plaques ELISA 96-puits à fond plats ayant une haute affinité de liaison pour les protéines, des anticorps anti HPBP sont adsorbés sur le support et incubée une nuit à 4°C. Les anticorps non fixés sur la plaque sont éliminés par lavage avec un tampon Phosphate Buffered Saline (PBS) contenant 10mg/mL de Bovine Serum Albumine (BSA).

Les puits sont saturés par une solution tampon Phosphate Buffered Saline (PBS) contenant 20mg/mL de Bovine Serum Albumine (BSA) pendant 1h à température ambiante afin d'éliminer le bruit de fond.

Après lavage avec une solution de Phosphate Buffered Saline (PBS) contenant 10mg/mL de Bovine Serum Albumine (BSA), différentes dilutions de sérum de patients sont ajoutées dans les puits pour une incubation 1-3h à RT.

La plaque est une nouvelle fois lavée puis incubée avec des anticorps anti-HPBP monoclonaux de souris ou des anticorps polyclonaux anti-HPBP de lapin pour une incubation de 1-3h à RT.

Finalement, après un nouveau lavage, des anticorps couplés à la Horseradish Peroxydase (HRP) reconnaissant les fragments Fc de souris ou de lapin sont ajoutées dans les puits pour une incubation 1-3h à RT.

Puis après un dernier lavage, la plaque est incubée avec un substrat de la HRP (la 3,3',5,5'-tétraméthylbenzidine : TNB en présence d'eau oxygénée H₂O₂). La réaction est arrêtée après 10 min par ajout de H₂SO₄. La densité optique du produit de la réaction est alors mesurée avec un spectrophotomètre à 450 nm.

L'ELISA permet de déterminer la concentration en protéine HPBP dans le sérum des patients.

### Exemple 13 : interaction in vitro des protéines HPBP et Tat

L'association de la Human Phosphate Binding Protein (HPBP) avec la protéine C/EBPβ est réalisée en utilisant une technique d'ELISA.

Dans des plaques ELISA 96-puits à fond plats ayant une haute affinité de liaison pour les protéines la protéine GST-Tat est adsorbée sur le support et incubée une nuit à 4°C.

Les protéines non fixées sur la plaque sont éliminées par lavage avec un tampon Phosphate Buffered Saline (PBS) contenant 10mg/mL de Bovine Serum Albumine (BSA).

Les puits sont saturés par une solution tampon Phosphate Buffered Saline (PBS) contenant 20mg/mL de Bovine Serum Albumine (BSA) pendant 1h à température ambiante afin d'éliminer le bruit de fond.

Après lavage grâce à une solution de Phosphate Buffered Saline (PBS) contenant 10mg/mL de Bovine Serum Albumine (BSA), différentes solutions de HPBP pure de 0.1nM à 100mM sont ajoutées dans les puits pour une incubation 1-3h à RT.

La plaque est une nouvelle fois lavée puis incubée avec des anticorps anti-HPBP monoclonaux de souris ou des anticorps polyclonaux anti-HPBP de lapin pour une incubation de 1-3h à RT.

Finalement, après un nouveau lavage, des anticorps couplés à la Horseradish Peroxydase (HRP) reconnaissant les fragments Fc de souris ou de lapin sont ajoutées dans les puits pour une incubation 1-3h à RT.

Puis après un dernier lavage, la plaque est incubée avec un substrat de la HRP (la 3,3',5,5'-tétraméthylbenzidine : TNB en présence d'eau oxygénée H₂O₂). La réaction est arrêtée après 10 min par ajout de H₂SO₄. La densité optique du produit de la réaction est alors mesurée avec un spectrophotomètre à 450 nm.

Les résultats montrent que la protéine HPBP et la protéine Tat sont capables d'interagir in vitro.

### Exemple 14 : Interaction in vitro des protéine HPBP et TAT

### Préalable :

Par immunisation de lapins et de souris, les Inventeurs ont obtenus des anticorps monoclonaux et polyclonaux ciblant la protéine HPBP. Les positions des épitopes reconnus par les anticorps monoclonaux ont été caractérisées et localisées sur la structure tridimensionnelle de HPBP. Ces outils moléculaires ont été utilisés afin de réaliser des études par western blots, immunohistochimie et ELISA. La connaissance de la position des épitopes reconnus par les différents anticorps monoclonaux a conduit à mettre en place un système permettant de quantifier et de cartographier les interactions entre HPBP et ses partenaires.

### Protocole :

Les inventeurs ont utilisé la technique de l'Enzyme Linked Immunosorbent Assay (ELISA), adaptée à l'étude de l'interaction entre la protéine HPBP et la protéine du VIH-1 (TAT) (Engvall and Perlmann, 1971)). La protéine TAT du VIH-1 a été exprimée sous forme d'une fusion avec la GST afin de l'obtenir la protéine soluble et en quantités (environs 2 mg/mL).

La protéine de fusion GST-TAT, diluée au 1/250 dans du PBS (Biorad ref : 161-0780EDU) est fixée par adsorption au fond des puits d'une plaque ELISA (Greiner Bio-one Médium Binding 96 Well Plate MICROLONTM 200 Flat-Bottom Clear) en laissant la plaque à 4°C sur la nuit. La plaque est ensuite saturée par une solution de Bovine Serum Albumine (BSA) à 20 mg/mL afin d'éliminer les sites de fixation non spécifiques pendant 1h à température ambiante puis lavée par du PBS. Trois dilutions de la protéine HPBP (1mg/mL, 0,5mg/mL et 0,25mg/mL) diluée dans du PBS/BSA à 10mg/mL sont ensuite déposées dans les puits pendant 3h à 37°C. La plaque est ensuite lavée 3 fois par du PBS/BSA à 10mg/mL afin d'éliminer les protéines non fixées. Afin de tester un épitope donné, l'anticorps monoclonal dilué au 1/1000ème dans du PBS/BSA 10mg/mL est déposé dans les puits pendant 1h à 37°C. La plaque est une nouvelle fois lavée 3 fois avec du PBS/BSA 10mg/mL afin d'éliminer les anticorps non fixés. Une dilution d'anticorps secondaires anti-souris Horseradish Peroxydase (Biorad ref : 170-5047) couplés à la dilués au 1/2000ème est ensuite déposée dans les puits afin de révéler l'interaction pendant 1h à 37°C. Finalement, la plaque est lavée 3 fois avec du PBS/BSA 10mg/mL puis 1 fois par du PBS et révélée en utilisant un substrat chromogénique (3,3',5,5'-tetramethylbenzidine (TMB), Biorad ref : 166-2402) qui révèle l'interaction.

En testant les capacités de fixation des différents anticorps monoclonaux, il est possible de mettre en évidence des épitopes qui ne sont plus reconnus. Ces épitopes sont masqués par la formation du complexe protéine DING - protéine cible. En identifiant les anticorps qui ne reconnaissent plus le complexe, nous pouvons modéliser sur la structure de HPBP la zone responsable de l'interaction.

Les régions reconnues par les anticorps sont représentés sur la figure 9A.

Dans le but d'obtenir une vision moléculaire de l'interaction en l'absence de données cristallographiques, les Inventeurs ont fait appel à la modélisation par docking moléculaire. Afin de modéliser l'interaction entre la protéine DING modèle HPBP et la protéine TAT, les Inventeurs ont utilisé la structure de la protéine TAT qui semble la plus précise (Anand et al., 2008, Nat Struct Mol Biol 15, 1287-1292). Le docking a été réalisé en utilisant le logiciel PATCHDOCK (Schneidman-Duhovny et al., 2005, Nucleic Acids Res 33, W363-367) dont les 100 meilleures solutions obtenues ont ensuite été injectées dans le logiciel FIREDOCK (Mashiach et al., 2008, Nucleic Acids Res 36, W229-232) qui permet d'affiner les résultats en libérant les chaines latérales. Nous avons ensuite obtenu les 10 meilleures solutions qui ont été classées par énergie globale et visualisées avec pymol 1.1. La solution la plus favorable en énergie globale, également contrastée par rapport aux autres solutions, est en accord avec les données expérimentales de la cartographie.

La modélisation de l'interaction HPBP/Tat est représentée sur la figure 9B.

Le tableau suivant récapitule les données d'interaction en précisant les épitopes de la protéine HPBP nécessaires pour l'interaction avec la protéine Tat

| **Séquences Pharmacophores** | **Données cartographiques (Mapping)** | **Données d'interaction (Docking)** |
|---|---|---|
| **1 - 6** | **DINGGG** | |
| **47 - 57** | **FGTDTTKNVHW** | |
| **78 - 80 78 - 80** | | **PGW** |
| **308 - 311** | | **ANAT** |
| **356 - 361** | **TASNAL** | |
| **370 - 376** | | **GGKGRPE** |

Ainsi une protéine capable d'interagir avec la protéine Tat est la suivante:

Les tirets (-) représentent l'un quelconque des 20 acides aminés naturels connus de l'état de la technique, ou éventuellement des acides aminés modifiés.

En d'autres termes, les acides aminés représentés par une lettre sont les acides aminés essentiels pour l'interaction. Aussi, tout variant de HPBP possédant une homologie, ou identité, d'au moins 72% par rapport à la protéine de séquence SEQ ID NO : 1, 2 ou 3, conservera ses propriétés d'interaction avec Tat si les acides aminés indiqués sont présents.

### Exemple 15 : Toxicité de la protéine HPBP

La toxicité cellulaire de la protéine HPBP a été mesurée sur des cellules en culture.

### Lignée cellulaire R3HR-1G5 (1g5)

5.10⁶ cellules/mL de la lignée lymphoïde B 1g5 ont été ensemencées dans des plaques et traitées ou non avec différentes doses de protéine HPBP purifié : 0,2 µg/mL, 2µg/mL, 20µg/mL, ou 90 µg/mL. Les cellules sont cultivées pendant 24 h à 37°C.

Après les 24h de culture, la toxicité de la protéine est évaluée en mesurant le nombre de cellules vivantes par la méthode MTT (bromure de 3-(4,5-dimethylthiazol-2-yl)-2,5-diphenyl tetrazolium) comme décrit dans Berridge et al. Biotechnology Annual review, 2005, 11, 127-152.

Brièvement, 10 µL de MTT est ajouté dans chaque culture.

Le mélange est ajouté vigoureusement pendant 1 minute, puis les cultures sont replacées à 37°C pendant 4h.

Le surnageant de culture est ensuite aspiré, et les cristaux formés par la présence de MTT sont dissous avec de l'acide acétique.

La densité optique (DO) de chacune des cultures est mesurée à 570 mn à l'aide d'un spectrophotomètre.

Les résultats obtenus sont présentés dans la figure 10.

Les résultats montrent que la dose létale 50% (DL50 - Dose tuant la moitié des cellules) est d'environ 20 µg/mL, ce qui correspond à une dose 10 à 100 fois supérieure à la dose nécessaire pour inhiber le VIH.

### Cellules Lymphocytaires de sang périphérique (PBL)

Des expériences similaires ont été réalisées sur des lymphocytes primaires. Les conditions similaires à celles utilisées pour les cellules 1g5 ont été utilisées.

Les cellules PBL ont été traitées avec 0,24 µg/mL, 0,48 µg/mL, 0,96 µg/mL, 2,4 µg/mL, 4,8 µg/mL, 9,6 µg/mL, ou 24 µg/mL de protéine HPBP. En contrôle des cellules non traitées ont été testées.

Les résultats sont présentés dans la figure 11.

Les résultats montrent que la DL50 est d'environ 5 µg/mL.

### Lignée cellulaire micro gliale

Des expériences similaires ont été réalisées sur des lignées microgliales. Les conditions similaires à celles utilisées pour les cellules 1g5 ont été utilisées, avec 5.10⁵ cellules/mL.

Les cellules PBL ont été traitées avec 0,24 µg/mL, 0,48 µg/mL, 0,96 µg/mL, 2,4 µg/mL, 4,8 µg/mL, 9,6 µg/mL, ou 24 µg/mL de protéine HPBP. En contrôle des cellules non traitées ont été testées.

Les résultats sont présentés dans la figure 12, courbe en pointillés.

Les résultats montrent que la DL50 est d'environ 2,5 µg/mL.

### Exemple 16 : Toxicité de la protéine p27SJ

La toxicité cellulaire de la protéine p27^{SJ} de millepertuis a été mesurée sur des cellules en culture dans les conditions décrites dans l'exemple 15. La p27^{SJ} a été testée à des doses de 0 µg/mL (contrôle), 0,01 µg/mL, 0,1 µg/mL, 0,5 µg/mL, 1 µg/mL, 5 µg/mL, 10 µg/mL, 20 µg/mL et 40 µg/mL.

Les résultats exprimés en pourcentage de toxicité sont présentés dans la figure 12, courbe en trait plein.

Les résultats montrent que la DL50 est nettement supérieure à 2,5 µg/mL (DL50 HPBP), puisqu'à une dose de 40 µg/mL de p27^{SJ}, seules environ 4% des cellules sont mortes. Ainsi la protéine p27^{SJ} a une toxicité très nettement inférieure à celle de la protéine HPBP.

Des résultats comparables à ceux obtenus pour la protéine p27^{SJ} ont été obtenus avec la protéine DING de pomme de terre.

### Exemples 17: Effet combiné HPBP + agents antiviraux

Méthode : une lignée cellulaire de Lymphocyte T CD4+ Jurkat (lignée 1G5) ayant intégré dans son génome une construction comprenant un gène rapporteur luciférase dont l'expression est contrôlée par le promoteur du VIH-1 (LTR du VIH-1; lignée Jurkat LTR-HIV) a été utilisée. La mesure d'une activité luciférase à partir des extraits cellulaires permet donc d'estimer l'activité transcriptionnelle du VIH-1.

Les lignées Jurkat LTR-HIV et Jurkat 1G5 ont été transfectées grâce à la technique du DEAE-dextran avec le génome proviral NL4.3 (génome VIH-1 complet, souche LAI).

Les lignées Jurkat LTR-HIV et Jurkat 1G5 ainsi infectées, sont traités 24 h plus tard avec la HPBP à une concentration finale de 2 µg/mL de milieu (52nM de protéine seule) ou HPBP 2 µg/mL + 10 µM AZT ou AZT seul. Les cellules et le surnageant cellulaire sont prélevés après 24h de traitement.

L'activité transcriptionnelle et l'inhibition de la réplication sont mesurées comme décrit dans l'exemple 6.

Les résultats indiquent que la combinaison HPBP + AZT exerce un meilleur effet sur l'activité transcriptionnelle et l'inhibition de la réplication.

Des résultats similaires sont obtenus avec des combinaisons HPBP+inhibiteur de transcriptase inverse, HPBP+inhibiteur d'entrée, HPBP+inhibiteur de fusion ou HPBP+inhibiteur d'intégrase.

## Revendications

1. Utilisation d'une composition comprenant ou constituée par au moins un élément choisi parmi :
- une protéine déterminée comprenant ou constituée de la séquence d'acides aminés choisie parmi les séquences suivantes SEQ ID NO: 5, SEQ ID NO: 6 et SEQ ID NO: 1 et
- une protéine homologue de ladite protéine déterminée, possédant une séquence telle que définie par une homologie d'au moins 72% avec une séquence choisie parmi les séquences suivantes SEQ ID NO : 5, SEQ ID NO : 6 et SEQ ID NO: 1, et interagissant avec le facteur de transcription C/EBPβ et/ou la protéine virale Tat,
pour
- la préparation d'un médicament destiné à la prévention ou au traitement d'une infection virale, ou
- pour la mise en oeuvre d'une méthode in vitro, de mesure la susceptibilité à une infection virale.

2. Utilisation d'une composition selon la revendication 1, où ladite protéine déterminée est constituée par une séquence d'acides aminés choisie parmi les séquences d'acides aminés suivantes SEQ ID NO: 5, SEQ ID NO : 7, SEQ ID NO: 9, SEQ ID NO: 6, SEQ ID NO: 8, SEQ ID NO : 10, SEQ ID NO : 1, SEQ ID NO : 2 et SEQ ID NO : 3.

3. Utilisation selon la revendication 1 ou 2, dans laquelle ladite infection virale est causée par un virus VIH, un virus de l'Hépatite ou un virus de l'Herpes.

4. Utilisation d'une composition selon la revendication 3, dans laquelle ledit virus du VIH est un virus VIH1 ou VIH2.

5. Utilisation selon l'une quelconque des revendications 1 à 4, dans laquelle ladite composition est administrée à une dose d'environ 0,1 nM à environ 10 µM, en particulier d'environ 10 nM à environ 100 nM, notamment d'environ 30nM à environ 70 nM, préférentiellement d'environ 50 nM.

6. Utilisation d'une composition selon l'une quelconque des revendications 1 à 5, en association avec au moins un composé possédant des propriétés antivirales et/ou avec au moins un composé possédant des propriétés anti inflammatoires.

7. Utilisation selon la revendication 6, dans laquelle ledit composé antiviral possédant des propriétés antivirales est choisi parmi les inhibiteurs d'antiprotéases, notamment ritonavir, indinavir, saquinavir et nelfinavir, et les inhibiteurs de transcriptase inverse, notamment l'AZT, ddI, ddC, 3TC, d4T, névirapine, delavirdine et efavirenz.

8. Utilisation d'une composition selon la revendication 6 ou 7, pour la préparation d'un médicament destiné à la prévention ou au traitement d'une infection virale,
la dite composition et ledit composé antiviral possédant des propriétés antivirales étant utilisés de manière simultanée, séparée ou étalée dans le temps.

9. Produit de combinaison comprenant :
- au moins un élément tel que défini dans la revendication 1 ou 2 , et
- au moins un composé possédant des propriétés antivirales, et éventuellement au moins un composé possédant des propriétés anti inflammatoires.

10. Composition pharmaceutique comprenant à titre de substance active au moins un élément choisi parmi
- une protéine déterminée comprenant ou constituée de la séquence d'acides aminés choisie parmi les séquences suivantes SEQ ID NO : 5, SEQ ID NO : 6 et SEQ ID NO: 1 et
- une protéine homologue de ladite protéine déterminée, possédant une séquence telle que définie par une homologie d'au moins 72% avec une séquence choisie parmi les séquences suivantes SEQ ID NO: 5, SEQ ID NO: 6 et SEQ ID NO: 1, et interagissant avec le facteur de transcription C/EBPβ et/ou la protéine virale Tat,
et au moins :
- composé antiviral possédant des propriétés antivirales, et éventuellement
- un composé possédant des propriétés anti inflammatoire,
en association avec un véhicule pharmaceutiquement acceptable.

11. Composition pharmaceutique selon la revendication 10, où ladite protéine déterminée est constituée par une séquence d'acides aminés choisie parmi les séquences d'acides aminés suivantes SEQ ID NO: 5, SEQ ID NO : 7, SEQ ID NO : 9, SEQ ID NO: 6, SEQ ID NO: 8, SEQ ID NO: 10, SEQ ID NO: 1, SEQ ID NO: 2 et SEQ ID NO: 3.

12. Méthode de diagnostic *in vitro* de la résistance à une infection par un virus, dans un échantillon biologique issu d'un individu, comprenant,
- la détermination dans ledit échantillon biologique de la quantité de protéine déterminée telle que définie dans la revendication 1 ou 2,
- la comparaison de ladite quantité obtenue à l'étape suivante avec la quantité de protéine déterminée d'au moins un échantillon de référence,
- la détermination, à partir de la comparaison précédente, de la résistance à une infection virale.

13. Méthode de diagnostic selon la revendication précédente, dans laquelle ladite infection est due à l'infection par les virus du VIH, notamment le virus VIH1 ou VIH2, les virus de l'Hépatite ou les virus de l'Herpes.

14. Composition pharmaceutique comprenant à titre de substance active au moins un élément choisi parmi
- une protéine déterminée **caractérisée en ce qu'**elle comprend une séquence d'acide aminée choisie parmi SEQ ID NO : 5, SEQ ID NO : 6 et SEQ ID NO: 1,
- une protéine homologue de ladite protéine déterminée, **caractérisée en ce qu'**elle possède une homologie d'au moins 72% avec -une séquence choisie parmi SEQ ID NO : 5, SEQ ID NO: 6 et SEQ ID NO: 1, et interagissant avec le facteur de transcription C/EBPβ et/ou la protéine virale Tat,
et au moins :
- composé antiviral possédant des propriétés antivirales, et/ou
- un composé possédant des propriétés anti inflammatoire
ledit possédant des propriétés antivirales étant notamment choisi parmi les inhibiteurs d'antiprotéases, tels que ritonavir, indinavir, saquinavir et nelfinavir, et les inhibiteurs de transcriptase inverse, tels que l'AZT, ddI, ddC, 3TC, d4T, névirapine, delavirdine et efavirenz,
en association avec un véhicule pharmaceutiquement acceptable,
pour la prévention ou le traitement d'une infection virale.

## Patentansprüche

1. Verwendung einer Zusammensetzung umfassend oder bestehend aus mindestens einem Element ausgewählt aus:
- einem bestimmten Protein umfassend die oder bestehend aus der Aminosäuresequenz ausgewählt aus den folgenden Sequenzen SEQ ID NO: 5, SEQ ID NO: 6 und SEQ ID NO: 1 und
- einem Protein, welches homolog ist zu dem bestimmten Protein, mit einer Sequenz definiert durch eine Homologie von mindestens 72 % mit einer Sequenz ausgewählt aus den folgenden Sequenzen SEQ ID NO: 5, SEQ ID NO: 6 und SEQ ID NO: 1, und welches mit dem Transkriptionsfaktor C/EBPβ und/oder dem viralen Protein Tat interagiert,
für
- die Herstellung eines Medikamentes, welches zur Prävention oder Behandlung einer viralen Infektion bestimmt ist, oder
- das Umsetzen eines in vitro Verfahrens der Messung der Anfälligkeit für eine virale Infektion.

2. Verwendung einer Zusammensetzung gemäß Anspruch 1, wobei das bestimmte Protein zusammengesetzt ist aus einer Aminosäuresequenz ausgewählt aus den folgenden Aminosäuresequenzen SEQ ID NO: 5, SEQ ID NO: 7, SEQ ID NO: 9, SEQ ID NO: 6, SEQ ID NO: 8, SEQ ID NO: 10, SEQ ID NO: 1, SEQ ID NO: 2 und SEQ ID NO: 3.

3. Verwendung einer Zusammensetzung gemäß Anspruch 1 oder 2, wobei die virale Infektion durch ein HIV-Virus, ein Hepatitis-Virus oder ein Herpes-Virus hervorgerufen wird.

4. Verwendung einer Zusammensetzung gemäß Anspruch 3, wobei das HIV-Virus ein HIV1- oder HIV2-Virus ist.

5. Verwendung gemäß einem der Ansprüche 1 bis 4, wobei die Zusammensetzung in einer Dosis von ungefähr 0,1 nM bis ungefähr 10 µM, insbesondere von ungefähr 10 nM bis ungefähr 100 nM, insbesondere von ungefähr 30 nM bis ungefähr 70 nM, vorzugsweise von ungefähr 50 nM verabreicht wird.

6. Verwendung einer Zusammensetzung gemäß einem der Ansprüche 1 bis 5, in Verbindung mit mindestens einer Verbindung mit antiviralen Eigenschaften und/oder mit mindestens einer Verbindung mit antiinflammatorischen Eigenschaften.

7. Verwendung gemäß Anspruch 6, wobei die antivirale Verbindung mit antiviralen Eigenschaften ausgewählt ist aus Antiprotease-Inhibitoren, insbesondere Ritonavir, Indinavir, Saquinavir und Nelfinavir, und Reverse Transkriptase Inhibitoren, insbesondere AZT, ddI, ddC, 3TC, d4T, Nevirapin, Delavirdin und Efavirenz.

8. Verwendung einer Zusammensetzung gemäß Anspruch 6 oder 7, zur Herstellung eines Medikamentes, welches bestimmt ist zur Prävention oder Behandlung einer viralen Infektion, wobei die Zusammensetzung und die antivirale Verbindung mit antiviralen Eigenschaften simultan, getrennt oder über die Zeit verteilt verwendet werden.

9. Kombinationsprodukt, umfassend:
- mindestens ein Element wie in Anspruch 1 oder 2 definiert, und
- mindestens eine Verbindung mit antiviralen Eigenschaften, und möglicherweise
- mindestens eine Verbindung mit antiinflammatorischen Eigenschaften.

10. Pharmazeutische Zusammensetzung umfassend als Wirkstoff mindestens ein Element ausgewählt aus
- einem bestimmten Protein umfassend die oder bestehend aus der Aminosäuresequenz ausgewählt aus den folgenden Sequenzen SEQ ID NO: 5, SEQ ID NO: 6 und SEQ ID NO: 1 und
- einem Protein, welches homolog ist zu dem bestimmten Protein, mit einer Sequenz definiert durch eine Homologie von mindestens 72 % mit einer Sequenz ausgewählt aus den folgenden Sequenzen SEQ ID NO: 5, SEQ ID NO: 6 und SEQ ID NO: 1, und welches mit dem Transkriptionsfaktor C/EBPβ und/oder dem viralen Protein Tat interagiert,
und mindestens:
- eine antivirale Verbindung mit antiviralen Eigenschaften, und möglicherweise
- eine Verbindung mit antiinflammatorischen Eigenschaften,
in Verbindung mit einem pharmazeutisch annehmbaren Träger.

11. Pharmazeutische Zusammensetzung gemäß Anspruch 10, wobei das bestimmte Protein aus einer Aminosäuresequenz ausgewählt aus den folgenden Aminosäuresequenzen SEQ ID NO: 5, SEQ ID NO: 7, SEQ ID NO: 9, SEQ ID NO: 6, SEQ ID NO: 8, SEQ ID NO: 10, SEQ ID NO: 1, SEQ ID NO: 2 und SEQ ID NO: 3 gebildet ist.

12. In-vitro-Diagnoseverfahren für die Resistenz gegen eine Infektion durch ein Virus, in einer biologischen Probe eines Individuums, umfassend
- die Bestimmung der Menge an bestimmtem Protein wie in Anspruch 1 oder 2 definiert in der biologischen Probe,
- den Vergleich der im nachfolgenden Schritt erhaltenen Menge mit der Menge an bestimmtem Protein mindestens einer Referenzprobe,
- die Bestimmung der Resistenz gegen eine virale Infektion anhand des vorhergehenden Vergleichs.

13. Diagnoseverfahren gemäß einem der voranstehenden Ansprüche, wobei die Infektion auf eine Infektion mit HIV-Viren, insbesondere dem HIV1- oder HIV2-Virus, Hepatitis-Viren oder Herpes-Viren zurückzuführen ist.

14. Pharmazeutische Zusammensetzung umfassend, als Wirkstoff, mindestens ein Element ausgewählt aus
- einem bestimmten Protein **gekennzeichnet dadurch, dass** es eine Aminosäuresequenz ausgewählt aus SEQ ID NO: 5, SEQ ID NO: 6 und SEQ ID NO: 1 umfasst,
- einem Protein, welches homolog ist zu dem bestimmten Protein, **gekennzeichnet dadurch, dass** es eine Homologie von mindestens 72 % mit einer Sequenz ausgewählt aus SEQ ID NO: 5, SEQ ID NO: 6 und SEQ ID NO: 1 besitzt, und welches mit dem Transkriptionsfaktor C/EBPβ und/oder dem viralen Protein Tat interagiert,
und mindestens:
- eine antivirale Verbindung mit antiviralen Eigenschaften, und/oder
- eine Verbindung mit antiinflammatorischen Eigenschaften
wobei die Verbindung mit antiviralen Eigenschaften insbesondere ausgewählt ist aus Antiprotease-Inhibitoren, wie Ritonavir, Indinavir, Saquinavir und Nelfinavir, und Reverse-Transkriptase-Inhibitoren, wie AZT, ddI, ddC, 3TC, d4T, Nevirapin, Delavirdin und
Efavirenz,
in Verbindung mit einem pharmazeutisch annehmbaren Träger,
für die Prävention oder die Behandlung einer viralen Infektion.

## Claims

1. Use of a composition comprising or constituted by at least one element chosen from:
- a specific protein comprising or constituted by the amino acid sequence selected among the following sequences SEQ ID NO: 5, SEQ ID NO: 6 and SEQ ID NO: 1, and
- a protein homologous to said specific protein, possessing an amino acid sequence exhibiting at least 72% homology with a sequence selected among the following sequences SEQ ID NO: 5, SEQ ID NO: 6 and SEQ ID NO: 1 and interacting with the transcription factor C/EBPβ and/or the viral protein Tat,
for
- the preparation of a medicament intended for the prevention or treatment of a viral infection, or
- the implementation of a method *in vitro,* for measuring susceptibility to a viral infection.

2. Use of a composition according to claim 1, where said specific protein is constituted by an amino acid sequence chosen from the following amino acid sequences: ID NO: 5, SEQ ID NO : 7, SEQ ID NO: 9, SEQ ID NO: 6, SEQ ID NO: 8, SEQ ID NO : 10, SEQ ID NO : 1, SEQ ID NO : 2 and SEQ ID NO : 3.

3. Use according to claim 1 or 2, in which said viral infection is caused by a HIV virus, a hepatitis virus or a herpes virus.

4. Use of a composition according to claim 3, in which said HIV virus is HIV-1 or HIV-2 virus.

5. Use according to any one of claims 1 to 4, in which said composition is administered at a dose from approximately 0.1 nm to approximately 10 µM, in particular from approximately 10 nM to approximately 100 nM, in particular from approximately 30 nM to approximately 70 nM, preferentially approximately 50 nM.

6. Use of a composition according to any one of claims 1 to 5, in combination with at least one compound possessing antiviral properties and/or with at least one compound possessing anti-inflammatory properties.

7. Use according to claim 6, in which said antiviral compound possessing antiviral properties is chosen from the antiprotease inhibitors, in particular ritonavir, indinavir, saquinavir and nelfinavir, and the reverse transcriptase inhibitors, in particular AZT, ddI, ddC, 3TC, d4T, nevirapine, delavirdine and efavirenz.

8. Use of a composition according to claim 6 or 7, for the preparation of a medicament intended for the prevention or treatment of a viral infection,
said composition and said antiviral compound possessing antiviral properties being used simultaneously, separately or spread over time.

9. Combination product comprising:
- at least one element according to cliam 1 or 2, and
- at least one compound possessing antiviral properties, and optionally one compound possessing anti-inflammatory properties.

10. Pharmaceutical composition comprising as active ingredient at least one element chosen from
- a specific protein comprising the amino acid sequence selected among the following sequences SEQ ID NO: 5, SEQ ID NO: 6 and SEQ ID NO: 1, and
- a protein homologous to said specific protein, possessing an amino acid sequence exhibiting at least 72% homology with a sequence selected among the following sequences SEQ ID NO: 5, SEQ ID NO: 6 and SEQ ID NO: 1 and interacting with the transcription factor C/EBPβ and/or the viral protein Tat,
and at least:
- one antiviral compound possessing antiviral properties, and optionally
- one compound possessing anti-inflammatory properties in combination with a pharmaceutically acceptable vehicle.

11. Pharmaceutical composition according to claim 10, where said specific protein is constituted by the amino acid sequence chosen from the following amino acid sequences: SEQ ID NO: 5, SEQ ID NO: 7, SEQ ID NO: 9, SEQ ID NO: 6, SEQ ID NO: 8, SEQ ID NO : 10, SEQ ID NO : 1, SEQ ID NO : 2 and SEQ ID NO : 3.

12. Method for *in vitro* diagnosis of resistance to infection with a virus, in a biological sample originating from an individual, comprising,
- the determination in said biological sample of the quantity of specific protein as defined in claim 1 or 2,
- the comparison of said quantity obtained in the following stage with the quantity of specific protein from at least one reference sample,
- the determination, from the previous comparison, of resistance to a viral infection.

13. Method of diagnosis according to the previous claim, in which said infection is due to infection with the HIV viruses, in particular the HIV-1 or HIV-2 viruses, hepatitis viruses or herpes viruses.

14. Pharmaceutical composition comprising as active ingredient at least one element chosen from
- a specific protein **characterized in that** it comprises an amino acid sequence SEQ ID NO: 5, SEQ ID NO: 6 and SEQ ID NO: 1, and
- a protein homologous to said specific protein, possessing an amino acid sequence exhibiting at least 72% homology with a sequence selected among the following sequences SEQ ID NO: 5, SEQ ID NO: 6 and SEQ ID NO: 1 and interacting with the transcription factor C/EBPβ and/or the viral protein Tat,
and at least:
- one antiviral compound possessing antiviral properties, and/or
- one compound possessing anti-inflammatory properties said compound possessing antiviral properties being in particular chosen from the antiprotease inhibitors, such as ritonavir, indinavir, saquinavir and nelfinavir, and the reverse transcriptase inhibitors, such as AZT, ddI, ddC, 3TC, d4T, nevirapine, delavirdine and efavirenz,
in combination with a pharmaceutically acceptable vehicle,
for the prevention or treatment of a viral infection.
